# EUROPÄISCHE PATENTSCHRIFT

(11) **EP 4 299 169 B1**
(45) Veröffentlichungstag und Bekanntmachung des Hinweises auf die Patenterteilung: **31.07.2024**
(21) Anmeldenummer: 22182103.6
(22) Anmeldetag: 30.06.2022
(51) Int. Cl.: B01F 33/501, B01F 35/71, B01F 35/75, B01F 31/441, B01F 101/20, A61B 17/88

(54) **VORRICHTUNG UND VERFAHREN ZUM BEREITSTELLEN VON KNOCHENZEMENTTEIG**
DEVICE AND METHOD FOR PRODUCING BONE CEMENT
DISPOSITIF ET PROCÉDÉ DE FOURNITURE DE LA PÂTE DE CIMENT OSSEUX

(43) Veröffentlichungstag der Anmeldung: 03.01.2024
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(56) Entgegenhaltungen:
- EP-A1- 3 150 155
- EP-A1- 3 260 192
- DE-A1- 102014 112 042

## Beschreibung

Die Erfindung betrifft eine Vorrichtung entsprechend Anspruch 1 zum Bereitstellen eines Knochenzementteigs aus zwei Ausgangskomponenten umfassend
eine Mischeinheit aufweisend eine hohlzylinderförmige Kartusche mit einem Innenraum, in dem ein Knochenzementpulver als erste Ausgangskomponente lagert, einem axial im Innenraum verschiebbaren Austragskolben, welcher den Innenraum an einem proximalen Kartuschenende fluidleitend verschließt, und einer Mischstange, welche über eine Mischstangendurchführung des Austragskolbens in den Innenraum geführt und beweglich im Innenraum gelagert ist, und ein Reservoir für eine Monomerflüssigkeit als zweite Ausgangskomponente umfassend ein Leitungsmittel, welches den Innenraum der Mischeinheit fluidleitend mit dem Reservoir verbindet,
wobei das Reservoir einen Reservoirbehälter, in dem mindestens eine fluidleitend geschlossene Ampulle mit einem Ampullenkörper und einem Ampullenkopf angeordnet ist und in der Ampulle die Monomerflüssigkeit lagert, und einen Hohlraum im Bereich des Ampullenkopfs aufweist,
wobei der Hohlraum fluidleitend mit dem Leitungsmittel verbunden ist und eine Verbindung zur Ampulle umfasst, wobei der Ampullenkopf zumindest bereichsweise in der Verbindung angeordnet ist und der Reservoirbehälter zumindest abschnittsweise einen verformbaren Bereich umfasst, so dass ein Verkippen der Ampulle um einen Drehpunkt gegen die Verbindung ermöglicht ist.

Die Erfindung betrifft weiterhin ein Verfahren entsprechend Anspruch 14 zum Bereitstellen eines Knochenzementteigs mit einer derartigen Vorrichtung.

### Hintergrund der Erfindung

Es werden erhebliche Bemühungen unternommen, Vorrichtungen und Verfahren zum Bereitstellen von Knochenzementteig aufzuzeigen, mittels derer Knochenzementteig einfach, zuverlässig und schnell bereitgestellt werden kann. Ein wichtiger Aspekt bei der Bereitstellung von Knochenzementteig ist die Vermeidung von Lufteinschlüssen, wie beispielsweise Gasblasen, im Knochenzement. Zu deren Vermeidung wurden eine Vielzahl von Vakuum-Zementiersystemen beschreiben, von denen exemplarisch folgende genannt sind:
US 6,033,105 A, US 5,624,184 A, US 4,671,263 A, US 4,973,168 A, US 5,100,241 A, WO 99/67015 A1, EP 1020167 A2, US 5,586,821 A, EP 1016452 A2, DE 3640279 A1, WO 94/26403 A1, EP 1005901 A2, EP 1886647 A1, US 5,344,232 A.

Eine Weiterentwicklung besteht in der Entwicklung von Zementiersystemen, in denen beide Ausgangskomponenten in separaten Bereichen der Mischsysteme gelagert sind und erst unmittelbar vor der Zementierapplikation im Zementiersystem miteinander vermischt werden. Solche geschlossenen, sogenannten Full-Prepacked-Systeme, sind in folgenden Schriften genannt: EP 0 692 229 A1, DE 10 2009 031 178 B3, US 5,997,544 A, US 6,709,149 B1, DE 698 12 726 T2, EP 0 796 653 A2, US 5,588,745 A.

In der WO 2012/038002 A1 wird eine Vorrichtung beschrieben, in der eine Monomerflüssigkeit aus einem Behälter über ein Leitungsmittel durch einen Kartuschenkopf in einen mit einem Knochenzementpulver gefüllten Innenraum einer Kartusche geleitet wird, so dass ein Knochenzementteig innerhalb der Kartusche durch Mischen bereitstellbar ist. Das Leitungsmittel und die Kartusche sind dabei über einen Formschluss miteinander verbunden. Nach einem Öffnen des Behälters, beispielsweise durch ein Öffnen eines Ventils, fließt die Monomerflüssigkeit über das Leitungsmittel in den Innenraum. Ein Nachteil des offenbarten Aufbaus der Vorrichtung ist, dass die Art des Öffnens des Behälters aufgrund der vergleichsweise instabilen Verbindung von Behälter zu Kartusche eingeschränkt sein kann. Beispielsweise könnte ein Öffnen des Behälters durch ein Verkippen der Ampulle zu einem Abknicken des Leitungsmittels führen.

Ein weiterer Nachteil der Vorrichtung ist der komplexe Aufbau des Kartuschenkopfs, da dieser sowohl für das Einleiten der Monomerflüssigkeit als auch für das Anmischen des Knochenzementteigs genutzt wird. Der Kartuschenkopf ist weiterhin über ein Gewinde mit der Kartusche verbunden, was die strukturelle Integrität der Vorrichtung schwächen könnte.

In der EP 2 404 864 A1 wird eine Vorrichtung zum Öffnen von Ampullen umfassend einen Außenbehälter, in dem eine fluidleitend geschlossene Ampulle mit einem Ampullenkörper und einem Ampullenkopf angeordnet ist, und einen Hohlraum beschrieben, wobei der Hohlraum eine Verbindung zu der Ampulle umfasst und der Ampullenkopf zumindest bereichsweise in der Verbindung angeordnet ist. Die Wände des Außenbehälters weisen wenigstens einen verformbaren Bereich auf, so dass ein Verkippen der Ampulle gegen die Verbindung ermöglicht ist, was die Ampulle fluidleitend öffnet.

In der EP 3 093 067 A1 wird eine Vorrichtung zum Bereitstellen eines Knochenzementteigs aus einer Monomerflüssigkeit und einem Knochenzementpulver beschrieben, welche eine Vorrichtung zum Öffnen von mit der Monomerflüssigkeit gefüllten Ampullen gemäß der

EP 2 404 864 A1 umfasst. Nach einem fluidleitenden Öffnen der Ampulle wird die Monomerflüssigkeit über ein Leitungsmittel durch eine Öffnung in einen mit dem Knochenzementpulver befüllten Innenraum einer Kartusche gefördert und dort mittels einer Mischeinrichtung, welche sich durch einen der Öffnung axial gegenüberliegenden Austragskolben erstreckt, mit dem Knochenzementpulver zu einem Knochenzementteig angemischt. Ein Nachteil der Vorrichtung ist, dass die beschriebene Vorrichtung aufgrund ihrer Funktionsweise einen vergleichsweise sperrigen Aufbau besitzt. Zudem verringert ein Einleiten der Monomerflüssigkeit auf einer Seite der Kartusche und ein Anmischen des Knochenzementteigs über eine hierzu axial gegenüberliegende Seite der Kartusche die Handlichkeit der Vorrichtung, was gleichzeitig mit einer Erhöhung der Komplexität der Vorrichtung als auch deren Handhabung einhergeht.

EP 3 150 155 A1 offenbart eine Vorrichtung entsprechend dem Oberbegriff des Anspruchs 1.

Es besteht im Markt der Wunsch zur weiteren Vereinfachung von Vorrichtungen zum Bereitstellen von Knochenzementteig.

### Aufgaben

Eine Aufgabe der vorliegenden Erfindung ist es, einen oder mehrere der sich aus dem Stand der Technik ergebenen Nachteile zumindest teilweise zu überwinden.

Im Speziellen basiert die Erfindung auf dem Ziel, eine Vorrichtung zur Verfügung zu stellen, welche ein einfaches und anwendungssicheres Öffnen einer oder mehrerer Ampullen, insbesondere Glasampullen, mit einer Monomerflüssigkeit zum einfachen, schnellen und anwendungssicheren Bereitstellen eines Knochenzementteigs erlaubt. Insbesondere soll das Öffnen der Ampulle oder der Ampullen mit möglichst wenig Aufwand und unter Vermeidung zusätzlicher, separater Werkzeuge erfolgen. Weiterhin soll das Öffnen der Ampulle mit möglichst wenigen Bauteilen ermöglicht sein. Weiterhin soll die Monomerflüssigkeit möglichst verlustfrei zum Bereitstellen des Knochenzementteigs zur Verfügung stellen. Ebenso soll die Vorrichtung eine möglichst hohe strukturelle Integrität bei möglichst kompakter Bauweise aufweisen.

Es ist eine weitere Aufgabe der Erfindung, ein Verfahren bereitzustellen, mit dem Knochenzement aus zwei Ausgangskomponenten bereitgestellt werden kann, mittels dem mindestens ein Teil der bereits beschriebenen Aufgaben zumindest zum Teil gelöst wird.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

Eine erste Ausführungsform der Erfindung ist eine Vorrichtung zum Bereitstellen eines Knochenzementteigs aus zwei Ausgangskomponenten umfassend
eine Mischeinheit aufweisend eine hohlzylinderförmige Kartusche mit einem Innenraum, in dem ein Knochenzementpulver als erste Ausgangskomponente lagert, einem axial im Innenraum verschiebbaren Austragskolben, welcher den Innenraum an einem proximalen Kartuschenende fluidleitend verschließt, und einer Mischstange, welche über eine Mischstangendurchführung des Austragskolbens in den Innenraum geführt und beweglich, insbesondere axial in Innenraum verschiebbar und um eine Längsachse der Mischstange drehbar, im Innenraum gelagert ist, und
ein Reservoir für eine Monomerflüssigkeit als zweite Ausgangskomponente umfassend ein Leitungsmittel, welches den Innenraum der Mischeinheit fluidleitend mit dem Reservoir verbindet,
wobei das Reservoir einen Reservoirbehälter, in dem mindestens eine fluidleitend geschlossene Ampulle mit einem Ampullenkörper und einem Ampullenkopf angeordnet ist und in der Ampulle die Monomerflüssigkeit lagert, und einen Hohlraum im Bereich des Ampullenkopfs aufweist,
wobei der Hohlraum fluidleitend mit dem Leitungsmittel verbunden ist und eine Verbindung zur Ampulle umfasst, wobei der Ampullenkopf zumindest bereichsweise in der Verbindung angeordnet ist und der Reservoirbehälter zumindest abschnittsweise einen verformbaren Bereich umfasst, so dass ein Verkippen der Ampulle um einen Drehpunkt gegen die Verbindung ermöglicht ist,
dadurch gekennzeichnet, dass
sich das Leitungsmittel durch eine Leitungsmitteldurchführung des Austragskolbens in den Innenraum erstreckt.

In einer Ausführungsform der Vorrichtung bilden das Leitungsmittel und die Leitungsmitteldurchführung einen ersten Formschluss, vorzugsweise einen reversiblen ersten Formschluss, zwischen der Mischeinheit und dem Reservoir aus. Diese Ausführungsform ist eine zweite Ausführungsform der Erfindung, welche vorzugsweise von der ersten Ausführungsform der Erfindung abhängt.

In einer Ausführungsform der Vorrichtung weist das Reservoir ein Verbindungselement auf, um das Reservoir über einen zweiten Formschluss, vorzugsweise über einen reversiblen zweiten Formschluss, mit der Mischeinheit zu verbinden. Diese Ausführungsform ist eine dritte Ausführungsform der Erfindung, welche vorzugsweise von der zweiten Ausführungsform der Erfindung abhängt.

In einer Ausführungsform der Vorrichtung ist das Verbindungselement eine Spange. Diese Ausführungsform ist eine vierte Ausführungsform der Erfindung, welche vorzugsweise von der dritten Ausführungsform der Erfindung abhängt.

In einer Ausführungsform der Vorrichtung bilden der Drehpunkt, der erste Formschluss und der zweite Formschluss in einer Seitenansicht die Eckpunkte eines Dreiecks aus. Diese Ausführungsform ist eine fünfte Ausführungsform der Erfindung, welche vorzugsweise von der dritten oder fünften Ausführungsform der Erfindung abhängt.

In einer Ausführungsform der Vorrichtung liegen der erste Formschluss, der zweite Formschluss und der Drehpunkt jeweils auf einer parallel zu einer Längsachse der Kartusche verlaufenden Geraden, wobei die Geraden einen unterschiedlichen Geradenabstand zur Längsachse der Kartusche aufweisen. Diese Ausführungsform ist eine sechste Ausführungsform der Erfindung, welche vorzugsweise von der dritten bis fünften Ausführungsform der Erfindung abhängt.

In einer Ausführungsform der Vorrichtung ist der zweite Formschluss zwischen dem Verbindungselement und der Mischstange ausgebildet. Diese Ausführungsform ist eine siebte Ausführungsform der Erfindung, welche vorzugsweise von der dritten bis sechsten Ausführungsform der Erfindung abhängt.

In einer Ausführungsform der Vorrichtung ist am Austragskolben ein Vakuumanschluss angeordnet, über den der Innenraum fluidleitend mit einer Unterdruckquelle, wie beispielsweise einer Vakuumpumpe, verbindbar ist. Diese Ausführungsform ist eine achte Ausführungsform der Erfindung, welche vorzugsweise von einer der vorhergehenden Ausführungsformen der Erfindung abhängt.

In einer Ausführungsform der Vorrichtung ist das Leitungsmittel ein Rohr, ein Schlauch oder ein Rohr und ein Schlauch. Diese Ausführungsform ist eine neunte Ausführungsform der Erfindung, welche vorzugsweise von einer der vorhergehenden Ausführungsformen der Erfindung abhängt.

In einer Ausführungsform der Vorrichtung ist das Leitungsmittel in der

Leitungsmitteldurchführung axial verschiebbar. Diese Ausführungsform ist eine zehnte Ausführungsform der Erfindung, welche vorzugsweise von der neunten Ausführungsform der Erfindung abhängt.

In einer Ausführungsform der Vorrichtung erstreckt sich das Leitungsmittel mindestens über 70 %, vorzugsweise mindestens über 80 %, weiter bevorzugt mindestens über 90 %, einer axialen Innenraumlänge des Innenraums. Diese Ausführungsform ist eine elfte Ausführungsform der Erfindung, welche vorzugsweise von der neunten oder zehnten Ausführungsform der Erfindung abhängt.

In einer Ausführungsform der Vorrichtung weist die Mischeinheit einen, vorzugsweise separat vorliegenden, Durchführungsverschluss auf, um die Leitungsmitteldurchführung nach einem Entfernen des Leitungsmittels fluidleitend zu verschließen. Diese Ausführungsform ist eine zwölfte Ausführungsform der Erfindung, welche vorzugsweise von einer der vorhergehenden Ausführungsformen der Erfindung abhängt.

In einer Ausführungsform der Vorrichtung weist die Kartusche an einem dem proximalen Kartuschenende axial gegenüberliegendem distalen Kartuschenende einen Kartuschenkopf auf, welcher das distale Kartuschenende fluidleitend verschließt, wobei der Kartuschenkopf eine Kartuschenkopfdurchführung zum fluidleitenden Verbinden eines Austragsschnorchels aufweist, über den der Knochenzementteig nach dem Bereitstellen aus dem Innenraum austragbar ist und wobei die Kartusche und der Kartuschenkopf einteilig ausgestaltet sind. Diese Ausführungsform ist eine dreizehnte Ausführungsform der Erfindung, welche vorzugsweise von einer der vorhergehenden Ausführungsformen der Erfindung abhängt.

Eine vierzehnte Ausführungsform der Erfindung ist ein Verfahren zum Bereitstellen eines Knochenzementteigs aus zwei Ausgangskomponenten mittels einer Vorrichtung nach einer der vorhergehenden Ausführungsformen der Erfindung, umfassend die Schritte:
a. Fluidleitendes Öffnen der mindestens einen Ampulle durch Verkippen der im Reservoirbehälter gelagerten Ampulle um den Drehpunkt gegen die Verbindung,
b. Fließen der Monomerflüssigkeit aus der mindestens einen Ampulle in den Hohlraum,
c. Fördern der Monomerflüssigkeit aus dem Hohlraum über das Leitungsmittel in den Innenraum.

In einer Ausführungsform des Verfahrens mittels einer Vorrichtung nach einer dritten bis dreizehnten Ausführungsformen der Erfindung umfasst das Verfahren zusätzlich die Schritte:
d. Lösen des zweiten Formschlusses,
e. Lösen des ersten Formschlusses durch Herausziehen des Leitungsmittels aus der Leitungsmitteldurchführung,
f. Fluidleitendes Verschließen der Leitungsmitteldurchführung,
g. Mischen von Knochenzementpulver und Monomerflüssigkeit.

Diese Ausführungsform ist eine fünfzehnte Ausführungsform der Erfindung, welche vorzugsweise von der vierzehnten Ausführungsform der Erfindung abhängt.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B. Ein "im Wesentlichen vollständiges Fördern einer Komponente C" umfasst beispielweise ein Fördern von ≥90 bis ≤100 Volumen-%, insbesondere ≥95 bis ≤100 Volumen-%, des Gesamtvolumens von C.

Die Begriffe "proximal" und "distal" dienen lediglich der Bezeichnung der räumlich entgegengesetzten Enden der Vorrichtung oder anderer Struktureinheiten der Vorrichtung und erlauben keinen Rückschluss auf die Orientierung in Bezug zu einem menschlichen Körper, beispielsweise eines Anwenders der Vorrichtung. "Distal zu ..." und "proximal zu..." oder ähnliche Formulierungen drücken entsprechend lediglich die räumliche Anordnung zweier Struktureinheiten der Vorrichtung zueinander aus.

### Ausführliche Beschreibung

Ein erster Gegenstand der Erfindung betrifft eine Vorrichtung zum Bereitstellen eines Knochenzementteigs aus zwei Ausgangskomponenten umfassend
eine Mischeinheit aufweisend eine hohlzylinderförmige Kartusche mit einem Innenraum, in dem ein Knochenzementpulver als erste Ausgangskomponente lagert, einem axial im Innenraum verschiebbaren Austragskolben, welcher den Innenraum an einem proximalen Kartuschenende fluidleitend verschließt, und einer Mischstange, welche über eine Mischstangendurchführung des Austragskolbens in den Innenraum geführt und beweglich, insbesondere axial im Innenraum verschiebbar und um eine Längsachse der Mischstange drehbar, im Innenraum gelagert ist, und
ein Reservoir für eine Monomerflüssigkeit als zweite Ausgangskomponente umfassend ein Leitungsmittel, welches den Innenraum der Mischeinheit fluidleitend mit dem Reservoir verbindet,
wobei das Reservoir einen Reservoirbehälter, in dem mindestens eine fluidleitend geschlossene Ampulle mit einem Ampullenkörper und einem Ampullenkopf angeordnet ist und in der Ampulle die Monomerflüssigkeit lagert, und einen Hohlraum im Bereich des Ampullenkopfs aufweist,
wobei der Hohlraum fluidleitend mit dem Leitungsmittel verbunden ist und eine Verbindung zur Ampulle umfasst, wobei der Ampullenkopf zumindest bereichsweise in der Verbindung angeordnet ist und der Reservoirbehälter zumindest abschnittsweise einen verformbaren Bereich umfasst, so dass ein Verkippen der Ampulle um einen Drehpunkt gegen die Verbindung ermöglicht ist,
dadurch gekennzeichnet, dass
sich das Leitungsmittel durch eine Leitungsmitteldurchführung des Austragskolbens in den Innenraum erstreckt.

Die Vorrichtung dient einem Anmischen eines Knochenzementteigs aus einem Knochenzementpulver und einer Monomerflüssigkeit, wobei vor dem Anmischen das Knochenzementpulver in einer Mischeinheit der Vorrichtung und die Monomerflüssigkeit in einem Reservoir der Vorrichtung lagert.

Das Reservoir dient dem Lagern der Monomerflüssigkeit, bevor diese durch Mischen mit dem Knochenzementpulver in der Mischeinheit den Knochenzementteig bereitstellt. Dazu weist das Reservoir einen Reservoirbehälter zur Aufnahme einer oder mehrerer, vorzugsweise zweier, mit der Monomerflüssigkeit befüllten fluidleitend geschlossenen Ampulle beziehungsweise Ampullen, insbesondere einer Glasampulle beziehungsweise Glasampullen, mit einem Ampullenkopf und einem Ampullenkörper auf. Der Reservoirbehälter umschließt die mindestens eine Ampulle, insbesondere zumindest den Ampullenkörper, so dass die Ampulle sicher im Reservoir bis zu ihrer Verwendung lagerbar ist. Der Reservoirbehälter kann beispielsweise in der Form eines Hohlzylinders vorliegen, in den die mindestens eine Ampulle eingeschoben ist, wobei zur verbesserten Transportfähigkeit der Vorrichtung der Reservoirbehälter so ausgeformt ist, beispielsweise indem der Reservoirbehälter einen Deckel aufweist, dass die Ampulle nicht unbeabsichtigt aus der Vorrichtung, insbesondere dem Reservoir, austreten kann. Vorzugsweise ist der Reservoirbehälter so ausgeformt, dass zwei Ampullen, insbesondere zwei Ampullen nebeneinander, vorzugsweise mit im Wesentlichen parallelen Längsachsen, in dem Reservoir gelagert werden können.

Weiterhin dient das Reservoir dem fluidleitenden Öffnen der mindestens einen Ampulle. Dazu weist das Reservoir einen Hohlraum auf, welcher über eine Verbindung mit der im Reservoirbehälter angeordneten Ampulle verbunden ist. Die Ampulle ist derart im Reservoir gelagert, dass der Ampullenkopf in Richtung des Hohlraums zeigt, während der Ampullenkörper zumindest anteilig, vorzugsweise vollständig, im Reservoirbehälter angeordnet ist. Zwischen Hohlraum und Reservoirbehälter erstreckt sich die Verbindung, und zwar derart, dass der Ampullenkopf zumindest bereichsweise in der Verbindung angeordnet ist. Dazu weist die Verbindung einen Verbindungsdurchmesser auf, welcher ein zumindest abschnittsweises Einschieben des Ampullenkopfs in die Verbindung erlaubt. In einer Ausgestaltungsform weist die Verbindung einen Verbindungsdurchmesser auf, welcher ein vollständiges Einschieben des Ampullenkopfs in die Verbindung erlaubt. Bevorzugt ist der Verbindungsdurchmesser kleiner als der Durchmesser des Ampullenkörpers, so dass dieser nicht in die Verbindung einschiebbar ist. Beispielsweise ist die Verbindung als Ring oder Hohlzylinder ausgestaltet und der Ampullenkopf ist zumindest bereichsweise von diesem Ring oder Hohlzylinder umgeben. Die Verbindung weist eine strukturelle Integrität auf, welche eine strukturelle Integrität der Ampulle übersteigt, so dass bei einem Pressen der Ampulle gegen die Verbindung die Ampulle zerbrechen kann. Um die Ampulle, oder bei der Anwesenheit von zwei oder mehr Ampullen alle Ampullen, zu öffnen, weist der Reservoirbehälter zumindest abschnittsweise, insbesondere angrenzend an einen Übergang des Ampullenkopfs zum Ampullenkörper der Ampulle, einen verformbaren Bereich auf. In einer Ausgestaltungsform ist der Reservoirbehälter vollständig verformbar. Der verformbare Bereich erlaubt ein Verkippen der Ampulle um einen Drehpunkt gegen die Verbindung. Dabei ist der Verbindungsdurchmesser so auf den Ampullenkopf abgestimmt, dass beim Verkippen zumindest das dem Ampullenkopf abgewandte Ampullenkörperende um den Drehpunkt verkippt wird, während zumindest das dem Ampullenkörper abgewandte Ampullenkopfende innerhalb der Verbindung verbleibt, so dass die Ampulle durch ein zumindest teilweises Zerbersten der Ampulle, insbesondere im Bereich eines Ampullenhalses zwischen Ampullenkopf und Ampullenkörper, fluidleitend geöffnet wird. Die Verbindung dient dabei hauptsächlich der Fixierung des Ampullenkopfs gegen eine Kippbewegung der Ampulle um den Drehpunkt. Beispielsweise ist der Verbindungsdurchmesser um nicht mehr als 10 % größer als der Durchmesser des Ampullenkopfs, so dass bereits ein relativ geringfügiges Verkippen der Ampulle zu deren fluidleitenden Öffnung führt.

Nach einem fluidleitenden Öffnen der zumindest einen Ampulle kann die Monomerflüssigkeit aus der Ampulle in den Hohlraum fließen. Der Hohlraum ist über ein Leitungsmittel fluidleitend mit der Mischeinheit, insbesondere mit einem Innenraum der Kartusche der Mischeinheit, in welchem das Knochenzementpulver lagert, verbunden. Ein Fördern der Monomerflüssigkeit aus dem Hohlraum über das Leitungsmittel in die Mischeinheit kann beispielsweise über die Schwerkraft, über einen Unterdruck in der Mischeinheit, insbesondere im Innenraum der Kartusche, oder einer Kombination davon ausgelöst werden.

Das Reservoir kann aus unterschiedlichsten Materialien oder Materialkombinationen bestehen. Beispiele kann das Reservoir aus einem Polymer bestehen. Bevorzugt handelt es sich bei dem Polymer um ein transparentes Polymer, da der Anwender auf diese Weise eine ordnungsgemäße Funktion des Reservoirs, insbesondere ein Ausfließen der Monomerflüssigkeit aus der zumindest einen Ampulle, während einer Verwendung optisch kontrollieren kann.

Die Mischeinheit dient einem Anmischen des Knochenzementteigs aus dem Knochenzementpulver und der Monomerflüssigkeit nach Fördern der Monomerflüssigkeit in die Mischeinheit, insbesondere in den Innenraum der Mischeinheit.

Die Mischeinheit weist eine hohlzylinderförmige Kartusche auf, in der das Knochenzementpulver gelagert ist. Unter einer hohlzylinderförmigen Kartusche ist ein rohrartiges Behältnis zu verstehen, welches einen Innenraum und eine den Innenraum umgebende Kartuschenwand aufweist. Der Querschnitt der Kartusche kann beliebige Formen annehmen. Aufgrund der einfachen Fertigung und der anwendungssichereren Verwendung der Vorrichtung ist der Querschnitt, und bevorzugt auch der Querschnitt des Innenraums, kreisförmig ausgestaltet. Dies erlaubt eine gute Handhabbarkeit für den Anwender und reduziert durch eine Abwesenheit von Kanten ein Risiko einer Verkeilung beweglicher Teile innerhalb der Vorrichtung.

Die Mischeinheit, insbesondere die Kartusche, kann aus unterschiedlichsten Materialien oder Materialkombinationen bestehen. Beispiele kann die Mischeinheit, insbesondere die Kartusche, aus einem Polymer bestehen. Bevorzugt handelt es sich bei dem Polymer um ein transparentes Polymer, da der Anwender auf diese Weise eine ordnungsgemäße Funktion der Mischeinheit während einer Verwendung optisch kontrollieren kann.

Die Mischeinheit weist einen im Innenraum axial verschiebbaren, vorzugsweise reversibel axial verschiebbaren, Austragskolben auf, welcher den Innenraum der Kartusche an einem proximalen Kartuschenende fluidleitend verschließt, so dass aus dem proximalen Kartuschenende kein Knochenzementpulver unbeabsichtigt austreten kann. Der Austragskolben ist so im Innenraum gelagert, dass dieser in Richtung des dem proximalen Kartuschenende axial gegenüberliegendem distalen Kartuschenende vortreibbar ist. Dies kann beispielsweise dazu dienen, den in der Vorrichtung, insbesondere im Innenraum der Mischeinheit, bereitgestellten Knochenzementteig aus dem distalen Kartuschenende auszutragen.

Der Austragskolben weist, vorzugsweise zentriert, eine axial durch den Austragskolben verlaufende Mischstangendurchführung auf, in welcher eine Mischstange angeordnet ist. Die Mischstange schließt dabei derart mit der Mischstangendurchführung ab, dass kein Knochenzementpulver oder Knochenzementteig aus der Mischstangendurchführung austreten kann.

Die Mischstange dient dem Anmischen von Knochenzementpulver und Monomerflüssigkeit unter Bereitstellung des Knochenzementteigs. Dazu erstreckt sich die Mischstange beweglich, insbesondere axial im Innenraum verschiebbar und vorzugsweise auch in der Mischstangendurchführung um die eigene Achse drehbar, durch die Mischstangendurchführung in den Innenraum hinein. Vorzugsweise weist die Mischstange eine Mischstangenlänge auf, welche länger als eine Innenraumlänge des Innenraums ist, so dass die Mischstange über die gesamte Innenraumlänge die Ausgangskomponenten mischen kann und gleichzeitig eine Bedienung der Mischstange durch einen Anwender von außerhalb der Mischeinheit erlaubt.

An einem distalen, in den Innenraum hineinragenden, Mischstangenende weist die Mischstange vorzugsweise ein Mischelement auf, um die Ausgangskomponenten möglichst über den gesamten Querschnitt des Innenraums gründlich durchmischen zu können. Beispielsweise ist das Mischelement als eine Mischscheibe mit mehreren, beispielsweise zwei bis sechs, axial durch die Mischscheibe verlaufenden Mischscheibendurchführungen ausgeformt. Die Mischstange weist vorzugsweise einen Durchmesser auf, welcher im Wesentlichen einem Durchmesser der Kartusche entspricht, was ein gründliches Anmischen der Ausgangskomponenten erleichtert.

Um die Bedienung von außerhalb der Mischeinheit für einen Anwender zu erleichtern, ist es bevorzugt, dass die Mischstange am proximalen, nicht im Innenraum gelagerten, Mischstangenende einen Griff aufweist.

Nach dem Bereitstellen des Knochenzementteigs wird die Mischstange zur weiteren Verwendung der Vorrichtung nicht mehr benötigt. In einer Ausgestaltungsform weist die Mischstange daher eine Sollbruchstelle auf, an welcher die Mischstange kontrolliert abbrechbar ist, so dass die nicht mehr aus dem Austragskolben herausragt. Vorzugsweise entspricht der Abstand der Sollbruchstelle vom distalen Mischstangenende ungefähr einer axialen Erstreckung des Austragskolbens. Dadurch liegt die Sollbruchstelle nach einem Herausziehen der Mischstange aus dem Innenraum bis zu einem distalen Anliegen des Mischelements am Austragskolben im Bereich der proximalen, dem Innenraum abgewandten, Austragskolbenseite, was ein im Wesentlichen vollständiges Abbrechen des aus der Mischeinheit ragenden Abschnitts der Mischstange erlaubt. Gleichzeitig ist dadurch das Mischelement direkt am Austragskolben angeordnet, so dass ein Vortreiben desselben in Richtung des distalen Kartuschenendes nicht durch die Mischstange und/oder das Mischelement behindert wird.

Die Mischeinheit ist mit dem Reservoir, insbesondere mit dem Hohlraum des Reservoirs, über das Leitungsmittel, welches sich durch eine Leitungsmitteldurchführung des Austragskolbens am proximalem Kartuschenende erstreckt, fluidleitend verbunden. Fluidleitend bedeutet, dass Flüssigkeiten, insbesondere die Monomerflüssigkeit, und Gase über das Leitungsmittel zwischen dem Reservoir und der Mischeinheit austauschbar sind. Vorzugsweise ist innerhalb des Leitungsmittels ein Filtermittel, insbesondere eine Porenscheibe, beispielsweise aus gesinterten Polypropylenpartikeln, aus gesinterten oder verpressten Polyethylenfasern, aus Cellulosefilz oder aus Pappe, angeordnet, welche das Leitungsmittel für Feststoffe undurchlässig ausgestaltet. Dieses verhindert, dass mögliche Bruchstücke der mindestens einen Ampulle in den Innenraum gelangen können. Bevorzugt ist das Filtermittel am dem Hohlraum gegenüberliegenden Ende des Leitungsmittels angeordnet, so dass kein Knochenzementpulver in das Leitungsmittel eindringen kann und so bei Kontakt mit der Monomerflüssigkeit unter Ausbildung des Knochenzementteigs das Leitungsmittel fluidleitend verschließen kann. Ein derartiges Filtermittel kann auch im Hohlraum angeordnet sein, um mögliche Bruchstücke der mindestens einen Ampulle bereits räumlich vor dem Leitungsmittel abzufangen.

Sowohl das Fördern der Monomerflüssigkeit als auch das Anmischen von Knochenzementpulver und der geförderten Monomerflüssigkeit wird räumlich durch den Austragskolben ermöglicht. Dies erlaubt einen möglichst einfachen und kompakten Aufbau der Vorrichtung. Gleichzeitig erlaubt diese weitgehende Konzentration der zum Bereitstellen des Knochenzementteigs benötigten Bauteile im Bereich des proximalen Kartuschenendes eine Vorrichtung mit einer möglichst hohen strukturellen Integrität, da andere Bereiche der Vorrichtung gezielt auf eine hohe Stabilität ausgerichtet werden können.

Eine Ausführungsform der Vorrichtung ist dadurch gekennzeichnet, dass das Leitungsmittel und die Leitungsmitteldurchführung einen ersten, vorzugsweise reversiblen, Formschluss zwischen der Mischeinheit und dem Reservoir ausbilden. Dies erlaubt ein anwendungssicheres und kontrolliertes Fördern der Monomerflüssigkeit aus dem Reservoir in den Innenraum der Mischeinheit für einen Anwender der Vorrichtung.

Eine Ausführungsform der Vorrichtung ist dadurch gekennzeichnet, dass das Reservoir ein Verbindungselement aufweist, um das Reservoir, vorzugsweise reversibel, über einen zweiten Formschluss mit der Mischeinheit zu verbinden.

In dieser Ausführungsform sind die Mischeinheit und das Reservoir über zumindest zwei Formschlüsse miteinander verbunden. Der erste Formschluss ist zwischen der Leitungsmitteldurchführung der Mischeinheit und dem Leitungsmittel des Reservoirs und der zweite Formschluss zwischen einem Verbindungselement des Reservoirs und der Mischeinheit ausgebildet.

Beim Öffnen der mindestens einen Ampulle durch ein Verkippen um den Drehpunkt gegen die Verbindung muss eine ausreichend hohe Kraft auf die Ampulle ausgeübt werden, um die strukturelle Integrität der Ampulle zu überwinden. Diese Kraft erhöht sich zusätzlich bei der Verwendung von mehr als einer Ampulle, wie vorzugsweise zwei Ampullen, falls diese, wie bevorzugt, gleichzeitig durch Verkippen um den Drehpunkt gegen die Verbindung geöffnet werden sollen. Diese Kraft wirkt auf die Kontaktstellen von Reservoir und Mischeinheit übertragen.

Falls das Reservoir und die Mischeinheit lediglich über den ersten Formschluss verbunden wären, würde die Kraft zum Öffnen der mindestens einen Ampulle vollständig auf das Leitungsmittel einwirken, so dass es zu einem Abknicken oder gar Abreißens des Leitungsmittels kommen könnte, wodurch ein im Wesentlichen vollständiges Fördern der Monomerflüssigkeit aus dem Reservoir in die Mischeinheit, insbesondere den Innenraum erschwert oder gar verhindert würde.

Der zweite Formschluss zwischen dem Verbindungselement und der Mischeinheit sorgt für eine Kraftverteilung der zum Öffnen der mindestens einen Ampulle benötigten Kraft auf die zwei Formschlüsse, so dass das Risiko eines Beschädigens der Vorrichtung beim Öffnen der mindestens einen Ampulle durch ein Verkippen verringert wird.

Durch die zwei Formschlüsse ist das Reservoir derart stabil mit der Mischeinheit verbunden, dass die mindestens eine Ampulle durch ein Verkippen um den Drehpunkt gegen die Verbindung geöffnet werden kann, ohne die Vorrichtung zu beschädigen und ohne zusätzliche Hilfsmittel neben der Vorrichtung zum Öffnen der mindestens einen Ampulle zu benötigen.

In einer Ausgestaltungsform ist das Verbindungselement einteilig mit dem restlichen Reservoir, insbesondere der Verbindung, dem Hohlraum und/oder dem Reservoirbehälter, verbunden. In einer weiteren Ausgestaltungsform ist das Verbindungsmittel lösbar mit dem restlichen Reservoir, insbesondere mit der Verbindung, dem Hohlraum und/oder dem Reservoirbehälter, verbindbar.

Die zwei Formschlüsse können so ausgestaltet sein, dass ein reversibles, insbesondere zerstörungsfreies, Trennen von Reservoir und Mischeinheit nicht möglich ist.

Eine Ausführungsform der Vorrichtung ist dadurch gekennzeichnet, dass der erste Formschluss und der zweite Formschluss lösbar ausgestaltet sind. Dies erlaubt ein einfaches, vorzugsweise reversibles, zerstörungsfreies Trennen von Reservoir und Mischeinheit, so dass nach dem Fördern der Monomerflüssigkeit aus der mindestens einen Kartusche durch das Leitungsmittel in den Innenraum der Kartusche das Reservoir von der Mischeinheit einfach getrennt werden kann. Da das Reservoir nach dem Fördern der Monomerflüssigkeit in die Mischeinheit nicht mehr benötigt wird, kann dies ein Bereitstellen des Knochenzementteigs für einen Anwender, beispielsweise durch eine verbesserte Handhabung der Mischeinheit, erleichtern.

Das Verbindungselement kann unterschiedlich ausgestaltet sein, um den zweiten Formschluss zwischen Reservoir und Mischeinheit auszubilden.

Eine Ausführungsform der Vorrichtung ist dadurch gekennzeichnet, dass das Verbindungselement eine Spange ist. Vorzugsweise ist die Spange aus zwei Spangeneinkerbungen an einer Reservoiraußenfläche, wie beispielsweise einer Außenfläche des Reservoirbehälters, des Hohlraums oder der Verbindung, sowie einem reversibel lösbaren Spangenelement gebildet, wobei das Spangenelement zwei Ausstülpungen aufweist, welche in die zwei Spangeneinkerbungen einschiebbar sind, so dass die Spange die Mischeinheit, vorzugsweise die Mischstange der Mischeinheit, manschettenartig umfasst und so den ersten Formschluss ausbildet. Dies erlaubt einen schnell und einfach erstell und lösbaren zweiten Formschluss, welcher stabil ist und ein sicheres Öffnen der mindestens einen Ampulle durch Verkippen um den Drehpunkt erlaubt.

Der Drehpunkt sowie der erste Formschluss und der zweite Formschluss können räumlich auf unterschiedliche Weise zueinander angeordnet sein.

Einer Ausführungsform der Vorrichtung ist dadurch gekennzeichnet, dass der Drehpunkt, der erste Formschluss und der zweite Formschluss in einer Seitenansicht, insbesondere einer Seitenansicht der Vorrichtung, die Eckpunkte eines Dreiecks ausbilden. In dieser Ausführungsform liegen der Drehpunkt und die beiden Formschlüsse in einer gemeinsamen Ebene, sind allerdings nicht auf einer in dieser Ebene verlaufenden Geraden angeordnet. Vorzugsweise liegt die Längsachse der Kartusche innerhalb dieser Ebene oder verläuft zumindest parallel zu dieser Ebene. Die Anordnung in Form eines Dreiecks verbessert die Kraftverteilung der zum fluidleitenden Öffnen der zumindest einen Ampulle benötigten Kraft, insbesondere bei einer dazu verwendeten Verkippbewegung der Ampulle um den Drehpunkt innerhalb oder parallel zu der Ebene des Dreiecks. Weiterhin ist durch eine derartige Anordnung der Drehpunkt in dieser Ebene fixiert und verschiebt sich nicht, was ein reproduzierbares Öffnen der mindestens einen Ampulle erleichtert.

Der Drehpunkt sowie der erste Formschluss und der zweite Formschluss können unterschiedlich große Abstände zueinander aufweisen.

Eine Ausführungsform der Vorrichtung ist dadurch gekennzeichnet, dass der zweite Formschluss einen kürzeren Abstand zum ersten Formschluss als der Drehpunkt aufweist. In dieser Ausführungsform ist der Abstand zwischen Drehpunkt und erstem Formschluss somit größer als der Abstand zwischen zweitem Formschluss und erstem Formschluss. Insbesondere bei einer Anordnung von Drehpunkt und den zwei Formschlüssen in Form eines Dreiecks erlaubt dies sowohl eine verbesserte Kraftverteilung der beim Verkippen zum Öffnen der Ampulle benötigten Kraft auf die zwei Formschlüsse als auch gleichzeitig eine möglichst platzsparende Ausgestaltung der Vorrichtung. Letzteres erleichtert insbesondere die Handhabung der Vorrichtung durch einen Anwender.

Vorzugsweise bilden dabei der Drehpunkt und die zwei Formschlüsse in einer Seitenansicht die Eckpunkte eines Dreiecks, wobei der Drehpunkt und der zweite Formschluss von den drei möglichen Abständen zwischen den genannten Punkten den geringsten Wert aufweisen. Dies führt zu einer weiteren Verbesserung der Kraftverteilung auf die zwei Formschlüsse und erlaubt eine weiter platzsparende Ausgestaltung der Vorrichtung.

Eine Ausführungsform der Vorrichtung ist dadurch gekennzeichnet, dass der erste Formschluss, der zweite Formschluss und der Drehpunkt jeweils auf einer parallel zu einer Längsachse der Kartusche verlaufenden Geraden liegen, wobei die Geraden einen unterschiedlichen Geradenabstand zur Längsachse der Kartusche aufweisen. Diese Anordnung verbessert die Kraftverteilung der beim Verkippen zum Öffnen der Ampulle benötigten Kraft auf die zwei Formschlüsse und erlaubt auch gleichzeitig eine möglichst platzsparende Ausgestaltung der Vorrichtung. Letzteres erleichtert insbesondere die Handhabung der Vorrichtung durch einen Anwender.

Vorzugsweise bilden dabei der Drehpunkt und die zwei Formschlüsse die Eckpunkte eines Dreiecks, wobei das Dreieck in einer Ebene liegt, in welcher auch die Längsachse der Kartusche liegt oder zu der die Längsachse der Kartusche zumindest parallel verläuft.

Der zweite Formschluss zwischen dem Reservoir, insbesondere zwischen dem Verbindungselement des Reservoirs, und der Mischeinheit kann an unterschiedlichen Stellen der Mischeinheit erfolgen.

Eine Ausführungsform der Vorrichtung ist dadurch gekennzeichnet, dass der zweite Formschluss zischen dem Verbindungselement und der Mischstange ausgebildet ist. Dies erleichtert ein Anbringen des Reservoirs auf der dem Innenraum gegenüberliegenden Seite des Austragskolbens und erleichtert somit ein Fördern der Monomerflüssigkeit durch das Leitungsmittel in den Innenraum. Letzteres wird insbesondere beim Ausnutzen der Schwerkraft zum Fördern der Monomerflüssigkeit erleichtert. Zudem erlaubt eine derartige Anordnung des zweiten Formschlusses eine möglichst einfache und platzsparende Ausgestaltung der Vorrichtung.

Um ein ungewolltes Vortreiben des Austragskolbens zu verhindern, kann am Austragskolben ein Rastmittel angeordnet sein, so dass der Austragskolben mit der Kartusche, insbesondere mit der Kartuschenwand, rastet, bis das Rastmittel aktiv von einem Anwender der Vorrichtung gelöst wird.

Eine Ausführungsform der Vorrichtung ist dadurch gekennzeichnet, dass am Austragskolben ein Vakuumanschluss angeordnet ist, über den der Innenraum fluidleitend mit einer Unterdruckquelle, wie beispielsweise einer Vakuumpumpe, verbindbar ist. Der Vakuumanschluss erlaubt somit ein Anlegen eines Unterdrucks im Innenraum, welcher beispielsweise zum Fördern der Monomerflüssigkeit aus dem Reservoir, insbesondere aus dem Hohlraum des Reservoirs, durch das Leitungsmittel in den Innenraum genutzt werden kann. Dies erlaubt ein einfaches und schnelles Fördern der Monomerflüssigkeit in den Innenraum. Weiterhin kann der Unterdruck im Innenraum während des Anmischens des Knochenzementteigs aus den beiden Ausgangskomponenten angelegt werden oder angelegt bleiben. Dadurch können Lufteinschlüsse im Knochenzementteig, welche sich nachteilig auf den ausgehärteten Knochenzement auswirken könnten, verringert oder vermieden werden.

Das Leitungsmittel kann unterschiedlich ausgestaltet sein, um die Monomerflüssigkeit in den Innenraum der Kartusche der Mischeinheit zu leiten.

Eine Ausführungsform der Vorrichtung ist dadurch gekennzeichnet, dass das Leitungsmittel ein Rohr, ein Schlauch oder eine Kombination von einem Rohr und einem Schlauch ist. Dies erlaubt eine einfache Herstellung sowie leichte und platzsparende Anordnung des Leitungsmittels innerhalb des Innenraums. Vorzugsweise ist das Leitungsmittel ein Rohr.

Es ist bevorzugt, dass das Leitungsmittel, insbesondere in Form eines Rohrs und/oder eines Schlauchs, einen fluidleitenden Leitungsmittelinnendurchmesser in einem Bereich von 0,5 mm bis 2 mm, bevorzugt in einem Bereich von 0,5 mm bis 1,5 mm aufweist. Kleinere Durchmesser verlangsamen das Fördern der Monomerflüssigkeit. Größere Durchmesser erschweren aufgrund geringerer Kapillareffekte im Leitungsmittel ein im Wesentlichen vollständiges Fördern der Monomerflüssigkeit.

Eine Ausführungsform der Vorrichtung ist dadurch gekennzeichnet, dass das Leitungsmittel in der Leitungsmitteldurchführung, vorzugsweise im Innenraum der Kartusche, axial verschiebbar ist. Dies erlaubt ein einfaches Lösen des ersten Formschlusses durch ein Herausziehen des Leitungsmittels aus der Leitungsmitteldurchführung. Vorzugsweise umgibt die Leitungsmitteldurchführung das Leitungsmittel manschettenartig, wobei das Leitungsmittel vorzugsweise ein Rohr ist. Dies stellt einen stabilen ersten Formschluss dar, welcher sich kontrolliert und einfach durch Herausziehen des Leitungsmittels aus der Leitungsmitteldurchführung lösen lässt. Um ein Lösen des ersten Formschlusses durch Herausziehen des Leitungsmittels aus der Leitungsmitteldurchführung zu erleichtern, ist es weiter bevorzugt, dass das Leitungsmittel ein biegsames Rohr ist.

Das Leitungsmittel erstreckt sich in oder durch die Leitungsmitteldurchführung und kann sich unterschiedlich weit in den Innenraum der Kartusche, insbesondere axial, erstrecken. In einer Ausgestaltungsform erstreckt sich das Leitungsmittel durch die Leitungsmitteldurchführung und schließt mit dieser auf Seite des Innenraums bündig ab. In dieser Ausgestaltungsform erstreckt sich das Leitungsmittel nicht innerhalb des Innenraums.

Eine Ausführungsform der Vorrichtung ist dadurch gekennzeichnet, dass sich das Leitungsmittel mindestens über 70 %, bevorzugt mindestens über 80 %, weiter bevorzugt mindestens über 90 %, der axialen Innenraumlänge des Innenraums erstreckt. Dies erlaubt, bei räumlicher Ausrichtung der Vorrichtung mit dem proximalen Kartuschenende nach oben und entsprechend dem distalen Kartuschenende nach unten, so das Knochenzementpulver in der Nähe des distalen Kartuschenendes lagert, ein Fördern der Monomerflüssigkeit direkt in das im Innenraum gelagerte Knochenzementpulver, welches vorzugsweise nicht den gesamten Innenraum der Kartusche, sondern beispielsweise lediglich 20-70 Volumen-% des gesamten Volumens des Innenraums, ausfüllt. Dabei ist bevorzugt, dass die Menge an Knochenzementpulver so gewählt ist, dass das Leitungsmittel die Monomerflüssigkeit in vertikaler Ausrichtung der Kartusche mit dem Kartuschenkopf nach oben zumindest auf eine Oberfläche des Knochenzementpulvers, bevorzugt zumindest 1 cm tief in das Knochenzementpulver leiten kann. Dies erleichtert ein Durchmischen der Ausgangskomponenten des Knochenzementteigs. Weiterhin wird dadurch die Monomerflüssigkeit in räumlicher Nähe des distalen Kartuschenendes im Innenraum, und so in vergleichsweise hoher räumlicher Distanz zum Vakuumanschluss am Austragskolben, falls vorhanden, freigesetzt. Dadurch wird vermieden, dass die Monomerflüssigkeit bei einem am Vakuumanschluss angelegten Unterdruck in den Vakuumanschluss eingesaugt wird und somit nicht zum Anmischen mit dem Knochenzementpulver zur Verfügung steht.

Eine Ausführungsform der Vorrichtung ist dadurch gekennzeichnet, dass die Mischeinheit einen Durchführungsverschluss aufweist, um die Leitungsmitteldurchführung nach einem Entfernen des Leitungsmittels, vorzugsweise durch Herausziehen des Leitungsmittels aus der Leitungsmitteldurchführung, fluidleitend zu verschließen. Der Durchführungsverschluss kann als separates Bauteil vorliegen oder als Teil des Austragskolbens oder der Kartusche ausgestaltet sein. In einer Ausgestaltungsform ist der Durchführungsverschluss als Stopfen ausgestaltet, welcher in die Leitungsmitteldurchführung einschiebbar, insbesondere von der proximalen Austragskolbenseite aus einschiebbar, ist. In einer weiteren Ausgestaltungsform ist der Durchführungsverschluss als ein Schieber ausgeformt, welcher durch ein Einschieben die Leitungsmitteldurchführung fluidleitend verschließt.

Durch den Durchführungsverschluss wird ein Anmischen des Knochenzements aus den Ausgangskomponenten innerhalb der Mischeinheit, insbesondere im Innenraum der Kartusche, mit angelegtem Unterdruck am Vakuumanschluss ermöglicht. Dadurch können Lufteinschlüsse im Knochenzementteig, welche sich nachteilig auf den ausgehärteten Knochenzement auswirken könnten, verringert oder vermieden werden.

Das distale Kartuschenende kann unterschiedlich ausgestaltet sein, um ein Bereitstellen des Knochenzementteigs im Innenraum zu ermöglichen. Beispielsweise kann das distale Kartuschenende mit einem Deckel fluidleitend verschlossen werden, bis der Knochenzementteig im Innenraum bereitgestellt wurde. Der Deckel weist hierzu einen Durchmesser auf, welcher im Wesentlichen einem Durchmesser des Innenraums entspricht, so dass der Deckel den Innenraum am distalen Kartuschenende vollständig fluidleitend verschließen kann. Dabei kann der Deckel beispielsweise über eine Gewindeverbindung oder über einen Bajonettverschluss mit der Kartusche, insbesondere reversibel, verbindbar sein. Nach einem Bereitstellen des Knochenzementteigs kann der Deckel vorzugsweise entfernt werden, um den Knochenzementteig aus dem Innenraum auszutragen.

Eine Ausführungsform der Vorrichtung ist dadurch gekennzeichnet, dass die Kartusche am distalen Kartuschenende einen Kartuschenkopf aufweist, welcher das distale Kartuschenende fluidleitend verschließt, wobei der Kartuschenkopf eine Kartuschenkopfdurchführung zum fluidleitenden Verbinden eines Austragsschnorchels aufweist, über den der Knochenzementteig aus dem Innenraum austragbar ist und wobei die Kartusche und der Kartuschenkopf einteilig ausgestaltet sind. Um eine Vorrichtung mit einer möglichst hohen strukturellen Integrität bereitzustellen, sind die Kartusche und der Kartuschenkopf einteilig ausgestaltet. Dies erspart ein Befestigen des Kartuschenkopfs, beispielsweise über eine Gewindeverbindung, an der Kartusche und verringert dadurch eine Schwächung der strukturellen Integrität der Vorrichtung. Der Kartuschenkopf weist eine Kartuschenkopfdurchführung auf, welche ein fluidleitendes Verbinden der Mischeinheit mit einem Austragsschnorchel, einem rohrartigen, länglichen Bauteil, zum kontrollierten Austragen des Knochenzements aus dem Innenraum erlaubt. Vorzugsweise weist die Kartuschenkopfdurchführung ein Innengewinde auf, welches mit einem Außengewinde eines Austragsschnorchels unter Ausbildung einer Gewindeverbindung zusammenschraubbar ist, um den Austragsschnorchel fluidleitend mit dem Innenraum zu verbinden. Die Kartuschenkopfdurchführung weist einen Durchmesser auf, welcher kleiner ist als der Durchmesser des Innenraums. Beispielsweise weist die Kartuschenkopfdurchführung einen Durchmesser auf, welcher 5-25 % des Durchmessers des Innenraums entspricht. Vorzugsweise ist die Kartuschenkopfdurchführung vor dem Anbringen eines Austragsschnorchels fluidleitend verschlossen, vorzugsweise über einen Kartuschenkopfverschluss mit einem Außengewinde, welcher form- und/oder kraftschlüssig mit einem Innengewinde der Kartuschenkopfdurchführung zusammenwirkt und so den Kartuschenkopfverschluss in der Kartuschenkopfdurchführung reversibel fixiert.

Ein weiterer Gegenstand der Erfindung betrifft ein Verfahren zum Bereitstellen eines Knochenzementteigs aus zwei Ausgangskomponenten mittels einer Vorrichtung nach einer der vorbeschriebenen Ausführungsformen der Erfindung, umfassend die Schritte:
a. Fluidleitendes Öffnen der mindestens einen Ampulle durch Verkippen der im Reservoirbehälter gelagerten Ampulle um den Drehpunkt gegen die Verbindung,
b. Fließen der Monomerflüssigkeit aus der mindestens einen Ampulle in den Hohlraum,
c. Fördern der Monomerflüssigkeit aus dem Hohlraum über das Leitungsmittel in den Innenraum.

In einem Schritt a. wird die mindestens eine Ampulle, wobei eine oder zwei Ampullen bevorzugt sind, durch Verkippen der im Reservoirbehälter gelagerten Ampulle um den Drehpunkt gegen die Verbindung fluidleitend geöffnet. Das Verkippen wird durch den zumindest abschnittsweisen verformbaren Bereich des Reservoirbehälters ermöglicht. Die mindestens eine Ampulle ist derart im Reservoir gelagert, dass der Ampullenkopf zumindest bereichsweise in der Verbindung des Reservoirs angeordnet ist, so dass beim Verkippen der Ampulle, insbesondere des Ampullenköpers im Reservoirbehälter, um den Drehpunkt der Ampullenkopf gegen die Verbindung gedrückt wird und so seine strukturelle Integrität überwunden wird, so dass die Monomerflüssigkeit aus der mindestens einen Ampulle ausfließen kann. Beim Öffnen bricht die Ampulle vorzugsweise im Bereich des Ampullenhalses zwischen Ampullenkopf und Ampullenkörper.

In einem Schritt b. fließt die Monomerflüssigkeit aus der in Schritt a. geöffneten Ampulle in den Hohlraum des Reservoirs aus. Um das Ausfließen aus der Ampulle zu erleichtern, wird dazu die Vorrichtung vorzugsweise so gehalten, dass die mindestens eine Ampulle einen Winkel zur Erdoberfläche in einem Bereich von 15-35 ° einnimmt. Je nach Ausgestaltung des Durchmessers der Verbindung kann beim Öffnen der Ampulle in Schritt a. der Ampullenkopf in den Hohlraum des Reservoirs fallen. Bevorzugt ist der Hohlraum so dimensioniert, dass der Ampullenkopf vollständig in den Hohlraum aufgenommen werden kann und zusätzlich der Ampullenkopf im Hohlraum rotierbar ist. So kann möglicherweise im Ampullenkopf vorhandene Monomerflüssigkeit gemäß der Schwerkraft in den Hohlraum ausfließen und steht somit zur Bereitstellung des Knochenzementteigs zur Verfügung.

In einem Schritt c. wird die Monomerflüssigkeit aus dem Hohlraum über das Leitungsmittel in den Innenraum gefördert. In einer Ausführungsform des Verfahrens wird die Schwerkraft zum Fördern der Monomerflüssigkeit in den Innenraum ausgenutzt. In einer weiteren, bevorzugten, Ausführungsform des Verfahrens wird das Fördern durch einen Unterdruck im Innenraum ausgelöst, wobei bevorzugt ist, dass der Unterdruck im Innenraum durch ein Anlegen eines Unterdrucks, beispielsweise durch fluidleitendes Verbinden des Vakuumanschlusses am Austragskolben mit einer Unterdruckquelle, wie beispielsweise einer Vakuumpumpe, erzeugt wird. In dieser Ausführungsform wird die Monomerflüssigkeit vorzugsweise mittels des Leitungsmittels direkt in das Knochenzementpulver eingeleitet, so dass ein Absaugen der Monomerflüssigkeit aus dem Innenraum durch Vakuumanschluss und in die Unterdruckquelle vermieden wird.

Eine Ausführungsform des Verfahrens ist dadurch gekennzeichnet, dass das Verfahren zusätzlich die folgenden Schritte umfasst:
d. Lösen des zweiten Formschlusses,
e. Lösen des ersten Formschlusses durch Herausziehen des Leitungsmittels aus der Leitungsmitteldurchführung,
f. Fluidleitendes Verschließen der Leitungsmitteldurchführung,
g. Mischen von Knochenzementpulver und Monomerflüssigkeit.

Diese Ausführungsform des Verfahrens wird mittels einer der vorstehend beschriebenen Ausführungsformen der Vorrichtung durchgeführt, welche das Verbindungselement zum Verbinden des Reservoirs mit der Mischeinheit über einen zweiten Formschluss aufweist.

Nachdem die Monomerflüssigkeit in die Mischeinheit gefördert wurde, besteht keine Notwendigkeit mehr das Reservoir mit der Mischeinheit über die zwei Formschlüsse verbunden zu lassen.

In einem Schritt d. wird der zweite Formschluss zwischen dem Reservoir, vorzugsweise zwischen dem Verbindungselement des Reservoirs, und der Mischeinheit, bevorzugt zwischen dem Verbindungselement und dem Mischstange der Mischeinheit, gelöst.

In einem Schritt e. wird der erste Formschluss durch Herausziehen des Leitungsmittels aus der Leitungsmitteldurchführung gelöst.

Die Schritte d. und e. können in beliebiger Reihenfolge durchgeführt werden, wobei es bevorzugt ist, erst Schritt d. und dann Schritt e. durchzuführen, da das Herausziehen des Leitungsmittels in Schritt e. erleichtert ist, wenn bereits der zweite Formschluss gelöst wurde.

In einem Schritt f. wird die Leitungsmitteldurchführung nach dem Herausziehen des Leitungsmittels fluidleitend verschlossen, so dass der Knochenzementteig bei angelegtem Unterdruck gemischt werden kann. Bevorzugt erfolgt das fluidleitende Verschließen der Kartuschenkopfdurchführung durch Verwendung des Durchführungsverschlusses.

In einem Schritt g. erfolgt ein Mischen des Knochenzementpulvers und der Monomerflüssigkeit unter Bereitstellen des Knochenzementteigs. Vorzugsweise erfolgt das Mischen mit angelegtem Unterdruck im Innenraum. Weiter bevorzugt erfolgt das Mischen unter Auf- und Abbewegen der Mischstange.

Der bereitgestellte Knochenzementteig kann auf unterschiedliche Weise aus der Vorrichtung ausgebracht werden. Beispielsweise kann der Knochenzementteig mit einem Spatel aus dem Innenraum herausgeholt werden.

Eine Ausführungsform des Verfahrens ist dadurch gekennzeichnet, dass nach dem Mischen des Knochenzementteigs ein Austragsschnorchel in einer Kartuschenkopfdurchführung am Kartuschenkopf der Mischeinheit fluidleitend angebracht wird und der Knochenzementteig durch Vortreiben des Austragskolbens in Richtung des Kartuschenkopfs durch den Austragsschnorchel ausgetragen wird. Dies erlaubt ein zielgerichtetes und kontrolliertes Austragen des Knochenzementteigs aus der Vorrichtung.

Vorzugsweise wird zum Austragen des Knochenzementteigs aus der Vorrichtung die Vorrichtung mit einer Austragshilfe, insbesondere einer Austragspistole, verbunden, welche den Austragskolben in Richtung des Kartuschenkopfs verschiebt und so den Knochenzementteig aus dem Innenraum austrägt.

Die Vorrichtung ist dadurch gekennzeichnet, dass diese einen Knochenzementteig aus zwei Ausgangskomponenten bereitstellt. Unter einem Knochenzementteig wird eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Durch Aushärtung wird aus einem Knochenzementteig ein Knochenzement. Bevorzugt handelt es sich bei diesen Knochenzementen um Polymethylmethacrylat-Knochenzemente (PMMA Knochenzemente). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzementteig. Dabei wird eine Polymerisation des Monomers durch Radikale eingeleitet. Mit fortschreitender Polymerisation des Monomers erhöht sich die Viskosität des Knochenzementteigs, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulver zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver als hydrophiles Additiv mindestens ein partikuläres Polymethylmethacrylat und/oder ein partikuläres Polymethylmethacrylat-Copolymer, einen Initiator und einen Röntgenopaker oder besteht aus diesen Komponenten.

Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin.

Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

Die für die Vorrichtung offenbarten Merkmale sind auch für das Verfahren offenbart und umgekehrt.

### Figuren

Die Erfindung wird im Folgenden durch Figuren weiter beispielhaft illustriert. Die Erfindung ist nicht auf die Figuren beschränkt.

Es zeigen
- Fig. 1: einen schematischen Längsschnitt einer Vorrichtung zum Bereitstellen eines Knochenzementteigs umfassend eine Mischeinheit und ein Reservoir mit einer mit einer Monomerflüssigkeit befüllten Ampulle,
- Fig. 2: eine perspektivische Seitenansicht eines Abschnitts der Vorrichtung aus Figur 1,
- Fig. 3: die Vorrichtung aus den Figuren 1 und 2, wobei ein erster Formschluss, ein zweiter Formschluss und ein Drehpunkt angedeutet sind,
- Fig. 4: die Vorrichtung aus den Figuren 1 bis 3 bei einem fluidleitenden Öffnen der Ampulle und beim Fördern der Monomerflüssigkeit in die Mischeinheit,
- Fig. 5: die Vorrichtung aus den Figuren 1 bis 54 wobei das Reservoir von der Mischeinheit getrennt ist und in der Mischeinheit der Knochenzementteig bereitgestellt wird,
- Fig. 6: die Vorrichtung aus den Figuren 1 bis 5 beim Austragen des Knochenzementteigs aus der Mischeinheit, und
- Fig. 7: ein Flussdiagramm eines Verfahrens zum Bereitstellen eines Knochenzementteigs.

### Beschreibung der Figuren

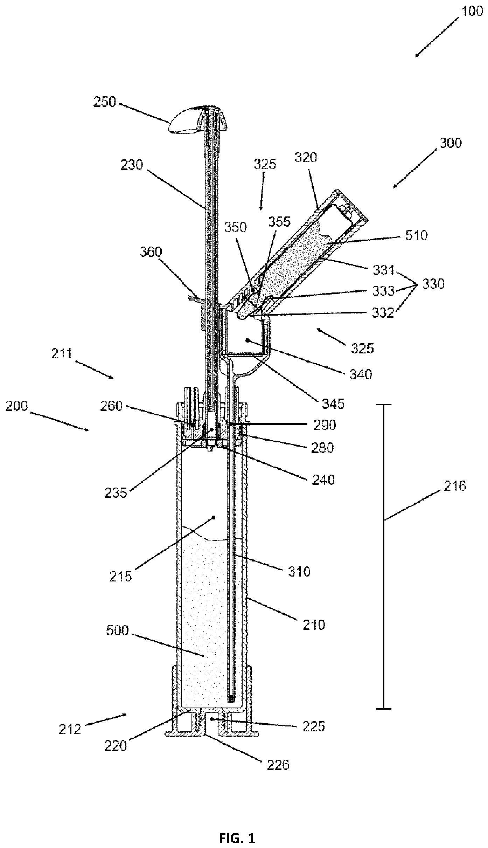

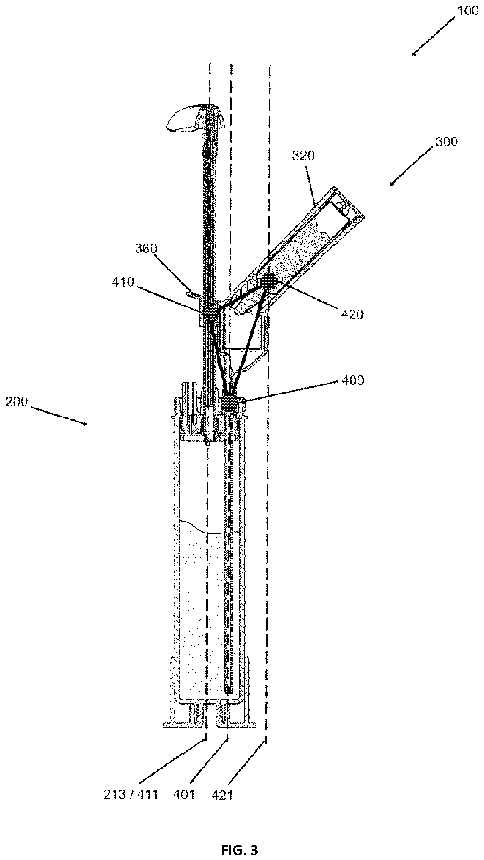

**Figur 1** zeigt einen schematischen Längsschnitt einer Seitenansicht einer beispielhaften Ausführung einer Vorrichtung 100 zum Bereitstellen eines Knochenzementteigs aus zwei Ausgangskomponenten in einem Ausgangszustand. Die Vorrichtung 100 umfasst eine Mischeinheit 200 und ein Reservoir 300, welche über zwei Formschlüsse miteinander verbunden sind (siehe auch Figur 3).

Die Mischeinheit 200 ist rohrartig aufgebaut und umfasst eine hohlzylinderförmige Kartusche 210 mit einem Innenraum 215, in welchem ein Knochenzementpulver 500 als eine erste Ausgangskomponente lagert. Ein proximales Kartuschenende 211 der Kartusche 210 ist mit einem axial im Innenraum 215 verschiebbaren Austragskolben 280 und ein dem proximalen Kartuschenende 211 axial gegenüberliegendes distales Kartuschenende 212 mit einem Kartuschenkopf 220 verschlossen. In der gezeigten Ausführungsform sind die Kartusche 210 und der Kartuschenkopf 220 einteilig ausgestaltet. Der Austragskolben 280 rastet reversibel am proximalen Kartuschenende 211, um ein unbeabsichtigtes Vortreiben desselben in Richtung des distalen Kartuschenendes 212 im Zuge des Bereitstellens des Knochenzementteigs zu unterbinden.

Der Kartuschenkopf 220 weist eine Kartuschenkopfdurchführung 225 auf, welche mit einem Kartuschenkopfverschluss 226 reversibel verschlossen ist. Dazu weist der Kartuschenkopf 220 im Bereich der Kartuschenkopfdurchführung 225 ein Innengewinde auf, welches form- und/oder kraftschlüssig mit einem Außengewinde des Kartuschenkopfverschlusses 226 ineinandergreift. Der Austragskolben 280 weist eine axial durch den Austragskolben 280 verlaufende Mischstangendurchführung 235 auf, in welcher axial verschiebbar und um sich selbst drehbar eine Mischstange 230 angeordnet ist. Die Mischstange 230 verschließt die Mischstangendurchführung 235, so dass kein Knochenzementpulver 500 unbeabsichtigt durch die Mischstangendurchführung 235 aus der Kartusche 210 austreten kann.

An einem dem Innenraum 215 abgewandten Ende der Mischstange 230 weist diese einen Griff 250 auf. Der Griff 250 dient der vereinfachten Handhabung der Vorrichtung 100 durch einen Anwender, indem er ein axiales Bewegen der Mischstange 230 innerhalb des Innenraums 215 erleichtert. An einem dem Griff 250 axial gegenüberliegendem ist die Mischstange 230 mit einem Mischelement 245 in Form einer Mischscheibe ausgestattet, welche ein Mischen des Knochenzementteigs im Innenraum 215 durch ein axiales Verschieben der Mischstange 230 innerhalb des Innenraums 215 erleichtert.

Am Austragskolben 280 ist ein Vakuumanschluss 260 angebracht, über welchen der Innenraum 215 fluidleitend mit einer Unterdruckquelle (nicht gezeigt) verbindbar ist.

Das Reservoir 300 weist einen rohrartigen Reservoirbehälter 320 auf, in welchem eine Ampulle 330, insbesondere eine Glasampulle, lagert. In weiteren, nicht gezeigten Ausführungsformen können im Reservoir 300 mehr als eine Ampulle 330, vorzugsweise zwei Ampullen 330 nebeneinander, gelagert werden. Die Ampulle 330 weist einen Ampullenkörper 331, einen der Mischeinheit 200 zugewandten Ampullenkopf 332 und einen zwischen Ampullenkörper 331 und Ampullenkopf 332 liegende Ampullenhals 333, welcher als Sollbruchstelle für die Ampulle 330 fungiert, auf. In der Ampulle 330 lagert eine Monomerflüssigkeit 510 als eine zweite Ausgangskomponente des Knochenzementteigs. Der Ampullenkopf 332 der Ampulle 330 ist abschnittsweise in einer Verbindung 350 angeordnet, welche einen Hohlraum 340 des Reservoirs 300 mit der Ampulle 330 verbindet. Die Verbindung 350 weist einen Verbindungsdurchmesser 355 auf, welcher etwa 5 % größer ist als ein Durchmesser des Ampullenkopfs 332, so dass die Verbindung 350 den Ampullenkopf 332 gegen ein Verkippen innerhalb der Zeichenebene fixiert. Um ein Verkippen der Ampulle 330, insbesondere des Ampullenkopfs 332, gegen die Verbindung 350, wobei in der gezeigten Ausführungsform ein Verkippen in der Zeicheneben möglich ist, zu ermöglichen, weist der Reservoirbehälter 320 im Bereich eines Übergangs von Verbindung 350 zu Ampullenkörper 331 einen verformbaren Bereich 325 auf.

Innerhalb des Hohlraums 340 ist im Reservoir 300 ein Filterelement 345 angeordnet, so dass Bruchstücke der Ampulle 330 nach einem fluidleitenden Öffnen derselben nicht über den Hohlraum 340 in die Mischeinheit 200 gelangen können, sondern am Filterelement 345 zurückgehalten werden.

Der Hohlraum 340 ist fluidleitend über ein Leitungsmittel 310 in Form eines Rohrs mit dem Innenraum 215 der Kartusche 210 verbunden. Das Leitungsmittel 310 erstreckt sich dazu durch eine Leitungsmitteldurchführung 290 des Austragskolbens 280 in den Innenraum 215 hinein. Das Leitungsmittel erstreckt sich circa über 95 % einer Innenraumlänge 216 des Innenraums 215, so dass bei der gezeigten Ausrichtung der Vorrichtung 100 die Monomerflüssigkeit 510 über das Leitungsmittel 310 direkt in das Knochenzementpulver 500 förderbar ist und einen genügend großen Abstand zum Vakuumanschluss 260 der Mischeinheit 200 aufweist, so dass die Gefahr eines direkten Absaugens der Monomerflüssigkeit 510 aus dem Leitungsmittel 310 durch den Vakuumanschluss 260 aus dem Innenraum 215 heraus verringert ist.

Die Leitungsmitteldurchführung 290 und das Leitungsmittel 310 bilden einen ersten Formschluss (vgl. auch Figur 3) aus, durch welchen die Mischeinheit 200 und das Reservoir 300 miteinander verbunden sind.

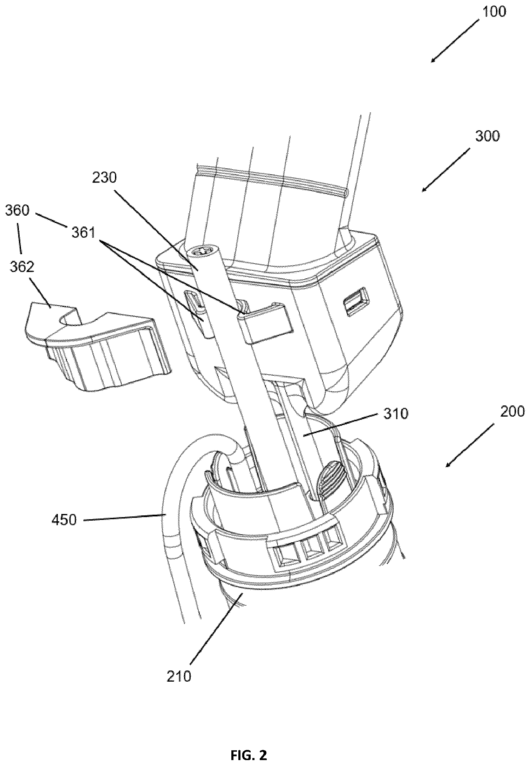

Das Reservoir 300 weist weiterhin ein Verbindungselement 360 in Form einer Spange auf (vgl. auch Figur 2), welches manschettenartig um die Mischstange 230 angeordnet ist und einen zweiten Formschluss (vgl. auch Figur 3) zwischen Mischeinheit 200 und Reservoir 300 ausbildet.

**Figur 2** zeigt eine schematische Seitenansicht eines Abschnitts der Vorrichtung 100 aus Figur 1. In Figur 2 ist ersichtlich, dass das Verbindungselement 360 aus zwei Spangeneinkerbungen 361 an einer Außenfläche des Reservoirs 300 und einem von den zwei Spangeneinkerbungen 361 lösbaren Spangenelement 362 gebildet ist. Das Spangenelement weist zwei Ausstülpungen (nicht gezeigt) auf, welche in die zwei Spangeneinkerbungen 261 eingeschoben werden können, so dass das Verbindungselement 360 manschettenartig um die Mischstange 230 reversibel schließbar ist, um den zweiten Formschluss stabil und sicher zwischen der Mischeinheit 200 und dem Reservoir 300 auszubilden. In der gezeigten Ausführungsform sind die zwei Spangeneinkerbungen 361 und die zwei Ausstülpungen des Spangenelements 362 dreieckig ausgestaltet, wobei ein Außendurchmesser der Ausstülpungen im Wesentlichen jeweils einem Innendurchmesser der Spangeneinkerbungen 261 entspricht.

In der gezeigten Ansicht ist am Vakuumanschluss 260 (nicht sichtbar) ein Schlauch 450 angeordnet, welcher die Vorrichtung 100 fluidleitend mit einer Unterdruckquelle (nicht gezeigt), wie beispielsweise einer Vakuumpumpe, verbindbar macht.

**Figur 3** zeigt die Vorrichtung 100 aus den Figuren 1 und 2, wobei der erste Formschluss 400, der zweite Formschluss 410 und ein Drehpunkt 420, um welchen die Ampullen 330 aufgrund des verformbaren Bereichs 325 durch Verkippen gegen die Verbindung 350 (vgl. Figur 1) drückbar sind, durch gefüllte Kreise angedeutet sind.

Der erste Formschluss 400, der zweite Formschluss 410 und der Drehpunkt 420 bilden in der gezeigten Seitenansicht der Vorrichtung 100 ein Dreieck (angedeutet durch Verbindungslinien zwischen den gefüllten Kreisen) aus. Erfolgt ein Verkippen der Ampullen 330 um den Drehpunkt 420, so dass die Ampullen 330, insbesondere die Ampullenköpfe 332 (vgl. Figur 1), gegen die Verbindung 350 (vgl. Figur 1) gedrückt werden, so verteilt sich die dabei zum fluidleitenden Öffnen der Ampullen 330 benötige Kraft auf den ersten Formschluss 400 und den zweiten Formschluss 410. Durch die Anordnung des ersten Formschlusses 400, des zweiten Formschlusses 410 und des Drehpunkts 420 in Form eines Dreiecks wird dabei eine vorteilhafte Kraftverteilung erreicht. Insbesondere kann dadurch die Gefahr eines Abknickens oder Abbrechens des Leitungsmittels 310 verringert werden.

Der erste Formschluss 400, der zweite Formschluss 410 und der Drehpunkt 420 sind so zueinander angeordnet, dass der zweite Formschluss 410 einen kürzeren Abstand zum ersten Formschluss 400 als der Drehpunkt 420 aufweist. Der Drehpunkt 420 und der erste Formschluss 400 sind somit weiter voneinander beabstandet als der erste Formschluss 400 und der zweite Formschluss 410. Dies sorgt für eine verbesserte Kraftverteilung der beim Verkippen zum Öffnen der Ampullen 330 um den Drehpunkt 420 benötigten Kraft auf die zwei Formschlüsse 400, 410 als auch gleichzeitig für eine möglichst platzsparende Ausgestaltung der Vorrichtung 100. Letzteres erleichtert insbesondere die Handhabung der Vorrichtung 100 durch einen Anwender.

Der erste Formschluss 400 liegt auf einer parallel zu einer Längsachse 213 der Kartusche 210 verlaufenden Geraden 401, der zweite Formschluss 410 liegt auf einer weiteren parallel zur Längsachse 213 der Kartusche 210 verlaufenden Geraden 411, wobei die Längsachse 213 und die Gerade 411 durch den zweiten Formschluss 410 im Wesentlichen deckungsgleich sind, und der Drehpunkt 420 liegt auf einer weiteren parallel zur Längsachse 213 der Kartusche 210 liegenden Geraden 421, wobei die Geraden 401, 411, 421 alle einen unterschiedlichen Geradenabstand zur Längsachse 213 der Kartusche 210 aufweisen. In der gezeigten Ausführungsform ist der Geradenabstand zwischen der Geraden 421 durch den Drehpunkt 420 und der Längsachse 213 am größten, gefolgt vom Geradenabstand zwischen der Geraden 401 durch den ersten Formschluss 400 und der Längsachse 213. Die unterschiedlichen Geradenabstände verbessern die Kraftverteilung der beim Verkippen zum Öffnen der Ampullen 330 um den Drehpunkt 420 benötigten Kraft auf die zwei Formschlüsse 400, 410 und erlauben gleichzeitig eine möglichst platzsparende Ausgestaltung der Vorrichtung 100. Letzteres erleichtert insbesondere die Handhabung der Vorrichtung 100 durch einen Anwender.

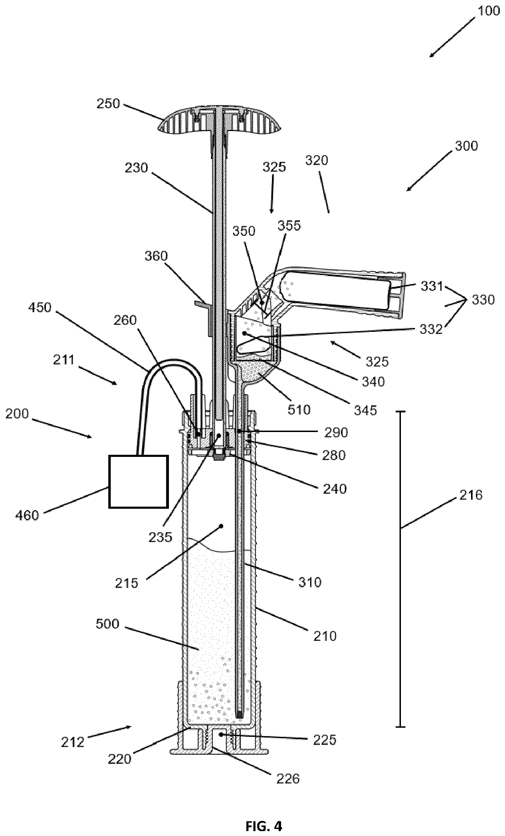

**Figur 4** zeigt die Vorrichtung 100 aus den Figuren 1 bis 3 mit um den Drehpunkt 420 (vgl. Figur 3) verkippter Ampulle 330. Dazu ist der Reservoirbehälter 320 am verformbaren Bereich 325 abgeknickt, so dass der Ampullenkopf 332 gegen die Verbindung 350 gepresst und die Ampulle 330 im Bereich des Ampullenhalses (vgl. Figur 1) fluidleitend geöffnet wurde. Die in der fluidleitend geöffneten Ampulle 330 gelagerte Monomerflüssigkeit 510 ist bereits größtenteils aus der Ampulle 330 in den Hohlraum 340 ausgeflossen. Der Ampullenkopf 332 ist vollständig aus der Verbindung 350 in den Hohlraum 3350 übergegangen. Dabei wurde der Ampullenkopf 332 vom Filterelement 345 aufgefangen, so dass dieser, oder Bruchstücke davon, nicht zum oder durch das Leitungsmittel 310 gelangen kann, beziehungsweise können. Der Hohlraum 340 ist so dimensioniert, dass der Ampullenkopf 332 vollständig drehbar in demselben lagerbar ist, so dass eventuell noch im Ampullenkopf 332 nach dem Öffnen der Ampulle 330 vorhandene Monomerflüssigkeit 510 in den Hohlraum 340 ausfließen kann. Dies ist in Figur 4 bei Ampullenkopf 332 bereits geschehen. Das Leitungsmittel 310, insbesondere ein Durchmesser des Leitungsmittels 310, ist in der gezeigten Ausführungsform der Vorrichtung 100 so ausgestaltet, dass die Monomerflüssigkeit 510 aufgrund deren Oberflächenspannung nicht allein aufgrund der Schwerkraft durch das Leitungsmittel 310 in den Innenraum 215 der Kartusche 210 fließen kann. Die aus den Ampullen 330 ausgeflossene Monomerflüssigkeit 510 sammelt sich daher zuerst im Hohlraum 340, bis aktiv von einem Anwender der Vorrichtung 100 eingegriffen wird. Um die Monomerflüssigkeit 510 aus dem Hohlraum 340 in den Innenraum 215 zu fördern, ist die Vorrichtung 100, insbesondere der Vakuumanschluss 260, fluidleitend über einen Schlauch 450 mit einer Unterdruckquelle 460 in Form einer Vakuumpumpe verbunden. Die Unterdruckquelle 460 erzeugt über den Schlauch 450 und den Vakuumanschluss 260 einen Unterdruck im Innenraum 215, welcher die Monomerflüssigkeit 510 aus dem Hohlraum 340 über das Leitungsmittel 310 in die Mischeinheit 200 saugt. Aufgrund der axialen Erstreckung des Leitungsmittels 310 wird die Monomerflüssigkeit 510 direkt in das Knochenzementpulver 500 eingeleitet, was die Gefahr eines Absaugens derselben durch den Vakuumanschluss 260 in die Unterdruckquelle 460 hinein verringert. Der Unterdruck im Innenraum 215 wird zumindest so lange aufrechterhalten, bis die Monomerflüssigkeit 510 im Wesentlichen vollständig in die Mischeinheit 200 überführt ist.

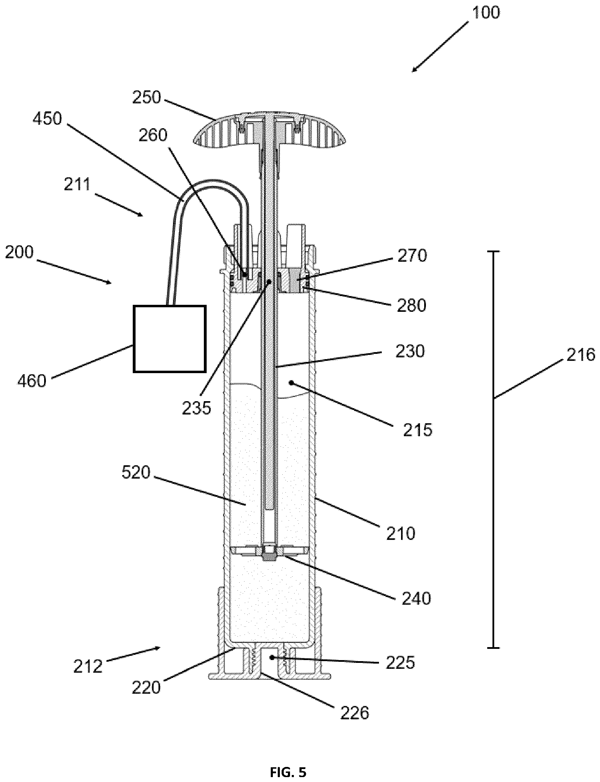

**Figur 5** zeigt die Vorrichtung 100 aus den Figuren 1 bis 4 beim Anmischen des Knochenzementteigs 520 aus den beiden Ausgangskomponenten im Innenraum 215 der Mischeinheit 200, wobei zur besseren Handhabbarkeit das Reservoir 300 vor dem Anmischen entfernt wurde. Zum Entfernen des Reservoirs 300 wurden der erste Formschluss 400 und der zweite Formschluss 410 zwischen Mischeinheit 200 und Reservoir 300 gelöst (vgl. Figuren 1 bis 4). Zum Lösen des zweiten Formschlusses 410 wurde das Spangenelement 362 aus den zwei Spangeneinkerbungen 261 (vgl. Figur 2) herausgezogen und zum Lösen des ersten Formschlusses 400 wurde das Leitungsmittel 310 aus der Leitungsmitteldurchführung 290 herausgezogen (vgl. Figuren 1 bis 4). Aufgrund einer einfacheren Handhabung, wurde dabei zuerst der zweite Formschluss 410 und dann der erste Formschluss 400 (vgl. Figur 3) gelöst. Nach dem Entfernen des Reservoirs 300 wurde die Leitungsmitteldurchführung 290 durch Eindrücken eines separaten Durchführungsverschlusses 270 in Form eines Stopfens fluidleitend verschlossen, so dass das Anmischen des Knochenzementteigs 520 mit im Innenraum 215 angelegtem Unterdruck stattfinden kann.

Zum Anmischen des Knochenzementteigs 520 wird die Mischstange 230 samt Mischelement 240 wiederholt im Innenraum 215 axial auf- und abbewegt.

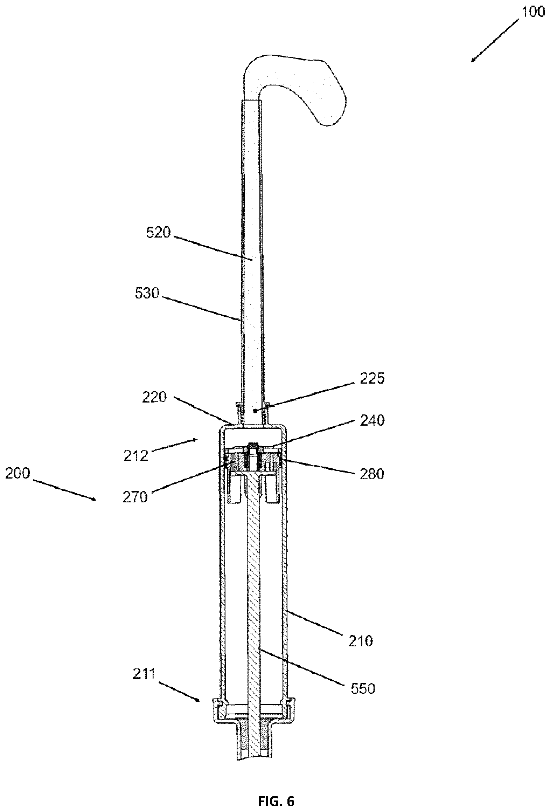

**Figur 6** zeigt die Vorrichtung 100 aus den Figuren 1 bis 5 beim Austragen des Knochenzementteigs 520. Dazu wurde der vormals am proximalen Kartuschenende 211 gerastete Austragskolben 280 gelöst und mittels einer Austragshilfe 550 in Form einer Austragspistole (lediglich abschnittsweise gezeigt) in Richtung des Kartuschenkopfs 220 am distalen Kartuschenende 212 vorgetrieben. Um das Vortreiben des Austragskolbens 280, insbesondere bei Verwendung einer Austragshilfe 550, zu erleichtern, wurde im Vorfeld die Mischstange 230 derart weit aus dem Innenraum 215 herausgezogen, bis das Mischelement 240 am Austragskolben 280 anliegt und im Anschluss der aus dem Austragskolben 230 herausragende Teil der Mischstange 230 samt Griff 250 an einer Sollbruchstelle im Bereich des Austragskolbens 280 abgebrochen.

Weiterhin wurde der Kartuschenkopfverschluss 226 entfernt und gegen einen Austragsschnorchel 530 ersetzt. Der Austragsschnorchel 530 weist dafür ein Außengewinde auf, welches form- und/oder kraftschlüssig mit dem Innengewinde des Kartuschenkopfs zusammenwirkt um den Austragsschnorchel 530 fluidleitend mit dem Innenraum 215 zu verbinden. Der Austragsschnorchel 530 erleichtert ein kontrolliertes und zielgenaues Austragen des Knochenzementteigs 530 aus der Vorrichtung 100.

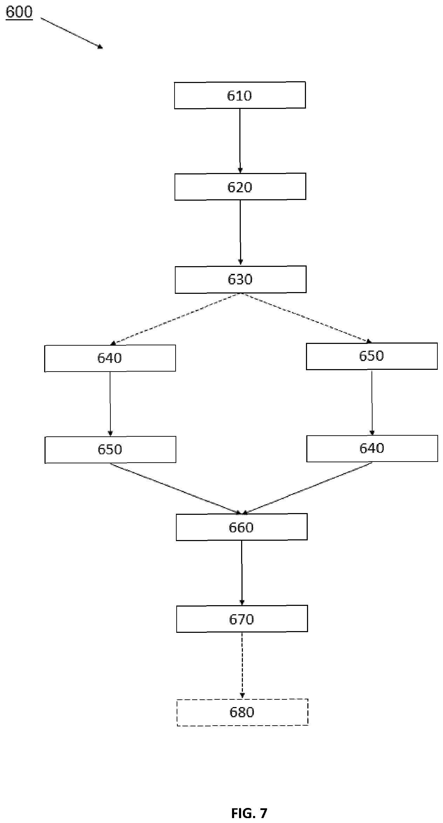

**Figur 7** zeigt ein Flussdiagramm eines Verfahrens 600 zur Bereitstellung eines Knochenzementteigs 520 aus zwei Ausgangskomponenten mittels einer Vorrichtung 100 gemäß den Figuren 1 bis 6 umfassend die Schritte 610 bis 630, vorzugsweise 610 bis 670 und optional auch Schritt 680.

In einem Schritt 610 wird die mindestens eine Ampulle 330 im Reservoir 300 durch Verkippen um den Drehpunkt 420 gegen die Verbindung 350 fluidleitend geöffnet. Die dafür benötigte Kraft wird vorzugsweise auf die zwei Formschlüsse 400, 410 verteilt, so dass ein Abknicken des Leitungsmittels 310 verhindert wird.

In einem Schritt 620 fließt die Monomerflüssigkeit 510 aus der mindestens einen in Schritt 610 fluidleitend geöffneten Ampulle 330 in den Hohlraum 340 des Reservoirs 300 aus. Vorzugsweise wird das Ausfließen der Monomerflüssigkeit 510 durch die Schwerkraft bewirkt. Bevorzugt werden beim Ausfließen der Monomerflüssigkeit 510 der Ampullenkopf 332, weitere oder andere Bruchstücke der mindestens einen Ampulle 332 durch ein Filterelement 345 im Hohlraum 340 zurückgehalten, so dass dieser oder diese nicht in das Leitungsmittel 310 vordringen können.

In einem Schritt 630 wird die Monomerflüssigkeit 510 aus dem Hohlraum 340 über das Leitungsmittel 310 in den Innenraum 215 der Kartusche 210 zum Knochenzementpulver 500 gefördert. In einer Ausführungsform wird zum Fördern der Monomerflüssigkeit 510 die Schwerkraft ausgenutzt. In einer weiteren Ausführungsform wird zum Fördern der Monomerflüssigkeit 510 ein im Innenraum 215 angelegter Unterdruck genutzt, welcher vorzugsweise über eine mit dem Vakuumanschluss 260 verbundene Unterdruckquelle 460, wie beispielsweise eine Vakuumpumpe, bereitgestellt wird. Vorzugsweise erfolgt das Fördern der Monomerflüssigkeit 510 direkt in das Knochenzementpulver 500 hinein, was eine bessere Durchmischbarkeit bewirken kann und gleichzeitig ein Risiko eines Absaugens der Monomerflüssigkeit 510 bei angelegtem Unterdruck über den Vakuumanschluss 260 aus dem Innenraum 215 heraus verringert oder verhindert.

Bevorzugt erfolgt in einem Schritt 640 ein Lösen des zweiten Formschlusses 410. Weiter bevorzugt ist das Verbindungselement 360 eine Spange, so dass zum Lösen des zweiten Formschlusses 410 das Spangenelement 362 aus den zwei Spangeneinkerbungen 361 gezogen wird.

Bevorzugt erfolgt in einem Schritt 650 ein Lösen des ersten Formschlusses 400 durch Herausziehen des Leitungsmittels 310 aus der Kartuschenkopfdurchführung 225.

Durch das Lösen 640, 650 der beiden Formschlüsse 400, 410 ist das Reservoir 300 von der Mischeinheit 200 getrennt und kann entfernt werden. Dies erleichtert die Handhabung der Mischeinheit 200.

Die zwei Formschlüsse 400, 410 können in beliebiger Reihenfolge oder gar gleichzeitig gelöst werden. In einer ersten Alternative erfolgt Schritt 640 zeitlich vor Schritt 650. In einer zweiten Alternative erfolgt Schritt 650 zeitliche vor Schritt 640.

Da vorzugsweise das Leitungsmittel 310 als Rohr ausgestaltet ist, welches sich im Innenraum 215 zumindest über 70 % der axialen Innenraumlänge 216 des Innenraums 215 erstreckt, ist aus Gründen der einfacheren Handhabung die erste Alternative bevorzugt.

Bevorzugt erfolgt in einem Schritt 660 ein fluidleitendes Verschließen der Kartuschenkopfdurchführung 225, insbesondere zeitlich nach dem Entfernen des Leitungsmittels 310 in Schritt 650. Dies erlaubt ein Anmischen von Knochenzementpulver 500 und Monomerflüssigkeit 510 unter Unterdruck, was Lufteinschlüsse im Knochenzementteig 520 verringern kann.

Vorzugsweise wird die Kartuschenkopfdurchführung 225 dabei mittels Durchführungsverschluss 270 verschlossen.

Bevorzugt erfolgt in einem Schritt 670 ein Mischen von Knochenzementpulver 500 und Monomerflüssigkeit 510 im Innenraum 215 der Kartusche 210 der Mischeinheit 200. Dazu ist es bevorzugt, dass in die Monomerflüssigkeit 510 mit dem Knochenzementpulver 500 mittels der Mischstange 230 bei angelegtem Unterdruck unter Ausbildung des Knochenzementteigs 520 vermischt wird. Bevorzugt ist die Mischstange 230 mit dem Mischelement 240, vorzugsweise in Form einer Mischscheibe ausgestattet, um das Anmischen zu erleichtern. Durch das Anmischen bei angelegtem Unterdruck verringern sich Lufteinschlüsse im bereitgestellten Knochenzementteig 520.

Um den bereitgestellten Knochenzementteig 520 aus der Vorrichtung 100 an eine gewünschte Stelle zu applizieren, ist es bevorzugt, dass in einem optionalen Schritt 680 die Mischstange 230 so weit aus der Mischstangendurchführung 235 aus dem Innenraum 215 herausgezogen, bis das Mischelement 240 distal am Austragskolben 280 anliegt, anschließend der proximal aus dem Austragskolben 280 herausragende Teil der Mischstange 230 an einer Sollbruchstelle abgebrochen und der Austragskolben 280 in Richtung des Kartuschenkopfverschlusses 220 unter Austragen des Knochenzementteigs aus der Vorrichtung 100 vorgetrieben. Bevorzugt wird das Vortreiben des Austragskolbens 280 in Richtung des Kartuschenkopfverschlusses 220 unter Hilfe einer Austragshilfe 550, wie beispielsweise einer Austragspistole, ausgelöst. Dies erleichtert dem Anwender die Verwendung der Vorrichtung 100.

### Bezugszeichen

- 100: Vorrichtung
- 200: Mischeinheit
- 210: hohlzylinderförmige Kartusche
- 211: proximales Kartuschenende
- 212: distales Kartuschenende
- 213: Längsachse der Kartusche
- 215: Innenraum der Kartusche
- 216: Innenraumlänge
- 220: Kartuschenkopf
- 225: Kartuschenkopfdurchführung
- 226: Kartuschenkopfverschluss
- 230: Mischstange
- 235: Mischstangendurchführung
- 240: Mischelement
- 250: Griff
- 260: Vakuumanschluss
- 270: Durchführungsverschluss
- 280: Austragskolben
- 290: Leitungsmitteldurchführung
- 300: Reservoir
- 310: Leitungsmittel
- 320: Reservoirbehälter
- 325: verformbarer Bereich
- 330: Ampulle
- 331: Ampullenkörper
- 332: Ampullenkopf
- 333: Ampullenhals
- 340: Hohlraum
- 345: Filterelement
- 350: Verbindung
- 355: Verbindungsdurchmesser
- 360: Verbindungselement
- 361: Spangeneinkerbungen
- 362: Spangenelement
- 400: erster Formschluss
- 401: Gerade durch ersten Formschluss
- 410: zweiter Formschluss
- 411: Gerade durch zweiten Formschluss
- 420: Drehpunkt
- 421: Gerade durch Drehpunkt
- 450: Schlauch
- 460: Unterdruckquelle
- 500: Knochenzementpulver
- 510: Monomerflüssigkeit
- 520: Knochenzementteig
- 530: Austragsschnorchel
- 550: Austragshilfe
- 600: Verfahren
- 610: fluidleitendes Öffnen
- 620: Fließen
- 630: Fördern
- 640: Lösen zweiter Formschluss
- 650: Lösen erster Formschluss
- 660: fluidleitendes Verschließen
- 670: Mischen
- 680: Austragen

## Patentansprüche

1. Vorrichtung (100) zum Bereitstellen eines Knochenzementteigs (520) aus zwei Ausgangskomponenten umfassend
eine Mischeinheit (200) aufweisend eine hohlzylinderförmige Kartusche (210) mit einem Innenraum (215), in dem ein Knochenzementpulver (500) als erste Ausgangskomponente lagert, einem axial im Innenraum (215) verschiebbaren Austragskolben (280), welcher den Innenraum (215) an einem proximalen Kartuschenende (211) fluidleitend verschließt, und einer Mischstange (230), welche über eine Mischstangendurchführung (235) des Austragskolbens (280) in den Innenraum (215) geführt und beweglich im Innenraum (215) gelagert ist, und
ein Reservoir (300) für eine Monomerflüssigkeit (510) als zweite Ausgangskomponente umfassend ein Leitungsmittel (310), welches den Innenraum (215) der Mischeinheit (200) fluidleitend mit dem Reservoir (300) verbindet,
wobei das Reservoir (300) einen Reservoirbehälter (320), in dem mindestens eine fluidleitend geschlossene Ampulle (330) mit einem Ampullenkörper (331) und einem Ampullenkopf (332) angeordnet ist und in der Ampulle (330) die Monomerflüssigkeit (510) lagert, und einen Hohlraum (340) im Bereich des Ampullenkopfs (332) aufweist,
wobei der Hohlraum (340) fluidleitend mit dem Leitungsmittel (310) verbunden ist und eine Verbindung (350) zur Ampulle (330) umfasst, wobei der Ampullenkopf (332) zumindest bereichsweise in der Verbindung (350) angeordnet ist und der Reservoirbehälter (320) zumindest abschnittsweise einen verformbaren Bereich (325) umfasst, so dass ein Verkippen der Ampulle (330) um einen Drehpunkt (420) gegen die Verbindung (350) ermöglicht ist,
**dadurch gekennzeichnet, dass**
sich das Leitungsmittel (310) durch eine Leitungsmitteldurchführung (290) des Austragskolbens (280) in den Innenraum (215) erstreckt.

2. Vorrichtung (100) nach Anspruch 1, **wobei** das Leitungsmittel (310) und die Leitungsmitteldurchführung (290) einen ersten Formschluss (400) zwischen der Mischeinheit (200) und dem Reservoir (300) ausbilden.

3. Vorrichtung (100) nach Anspruch 2, **wobei** das Reservoir (300) ein Verbindungselement (360) aufweist, um das Reservoir (300) über einen zweiten Formschluss (410) mit der Mischeinheit (200) zu verbinden.

4. Vorrichtung (100) nach Anspruch 3, **wobei** das Verbindungselement (360) eine Spange ist.

5. Vorrichtung (100) nach Anspruch 3 oder 4, **wobei** der Drehpunkt (420), der erste Formschluss (400) und der zweite Formschluss (410) in einer Seitenansicht die Eckpunkte eines Dreiecks ausbilden.

6. Vorrichtung (100) nach einem der Ansprüche 3 bis 5, **wobei** der erste Formschluss (400), der zweite Formschluss (410) und der Drehpunkt (420) jeweils auf einer parallel zu einer Längsachse (213) der Kartusche (210) verlaufenden Geraden (401, 411, 421) liegen, wobei die Geraden (401, 411, 421) einen unterschiedlichen Geradenabstand zur Längsachse (213) der Kartusche (210) aufweisen.

7. Vorrichtung (100) nach einem der Ansprüche 3 bis 6, **wobei** der zweite Formschluss (410) zwischen dem Verbindungselement (360) und der Mischstange (230) ausgebildet ist.

8. Vorrichtung (100) nach einem der vorhergehenden Ansprüche, **wobei** am Austragskolben (280) ein Vakuumanschluss (260) angeordnet ist, über den der Innenraum (215) fluidleitend mit einer Unterdruckquelle verbindbar ist.

9. Vorrichtung (100) nach einem der vorhergehenden Ansprüche, **wobei** das Leitungsmittel (310) ein Rohr und/oder ein Schlauch ist.

10. Vorrichtung (100) nach Anspruch 9, **wobei** das Leitungsmittel (310) in der Leitungsmitteldurchführung (290) axial verschiebbar ist.

11. Vorrichtung (100) nach Anspruch 9 oder 10, **wobei** sich das Leitungsmittel (310) mindestens über 70 % einer axialen Innenraumlänge (216) des Innenraums (215) erstreckt.

12. Vorrichtung (100) nach einem der vorhergehenden Ansprüche, **wobei** die Mischeinheit (200) einen Durchführungsverschluss (270) aufweist, um die Leitungsmitteldurchführung (290) nach einem Entfernen des Leitungsmittels (310) fluidleitend zu verschließen.

13. Vorrichtung (100) nach einem der vorhergehenden Ansprüche, **wobei** die Kartusche (210) an einem dem proximalen Kartuschenende (211) axial gegenüberliegendem distalen Kartuschenende (212) einen Kartuschenkopf (220) aufweist, welcher das distale Kartuschenende (212) fluidleitend verschließt, wobei der Kartuschenkopf (220) eine Kartuschenkopfdurchführung (225) zum fluidleitenden Verbinden eines Austragsschnorchels (530) aufweist, über den der Knochenzementteig (520) nach dem Bereitstellen aus dem Innenraum (215) austragbar ist und wobei die Kartusche (210) und der Kartuschenkopf (220) einteilig ausgestaltet sind.

14. Verfahren (600) zum Bereitstellen eines Knochenzementteigs (520) aus zwei Ausgangskomponenten mittels einer Vorrichtung (100) nach einem der vorhergehenden Ansprüche, umfassend die Schritte:
a. Fluidleitendes Öffnen (610) der mindestens einen Ampulle (330) durch Verkippen der im Reservoirbehälter (320) gelagerten Ampulle (330) um den Drehpunkt (420) gegen die Verbindung (350),
b. Fließen (620) der Monomerflüssigkeit (510) aus der mindestens einen Ampulle (330) in den Hohlraum (340),
c. Fördern (630) der Monomerflüssigkeit (510) aus dem Hohlraum (340) über das Leitungsmittel (310) in den Innenraum (215).

15. Verfahren (600) nach Anspruch 14 mittels einer Vorrichtung (100) nach einem der Ansprüche 3 bis 13 zusätzlich umfassend die Schritte:
d. Lösen (640) des zweiten Formschlusses (410),
e. Lösen (650) des ersten Formschlusses (400) durch Herausziehen des Leitungsmittels (310) aus der Leitungsmitteldurchführung (290),
f. Fluidleitendes Verschließen (660) der Leitungsmitteldurchführung (290),
g. Mischen (670) von Knochenzementpulver (500) und Monomerflüssigkeit (510).

## Claims

1. A device (100) for preparing a bone cement paste (520) consisting of two starting components, comprising
a mixing unit (200) comprising a hollow-cylindrical cartridge (210) that has an interior (215) in which a bone cement powder (500) is stored as a first starting component, a dispensing plunger (280) which is axially displaceable in the interior (215) and fluid-conductingly seals the interior (215) at a proximal cartridge end (211), and a mixing rod (230) which is guided into the interior (215) via a mixing rod duct (235) of the dispensing plunger (280) and is movably mounted in the interior (215), and
a reservoir (300) for a monomer liquid (510) as a second starting component, comprising a conduit means (310) which fluid-conductingly connects the interior (215) of the mixing unit (200) to the reservoir (300),
wherein the reservoir (300) comprises a reservoir container (320), in which at least one fluid-conductingly closed ampoule (330) having an ampoule body (331) and an ampoule head (332) is arranged, and the monomer liquid (510) is stored in the ampoule (330), and a cavity (340) in the region of the ampoule head (332), wherein the cavity (340) is fluid-conductingly connected to the conduit means (310) and comprises a connection (350) to the ampoule (330), wherein the ampoule head (332) is arranged at least partly in the connection (350) and the reservoir container (320) comprises, at least in part, a deformable region (325), so that the ampoule (330) can be tilted about a pivot point (420) against the connection (350),
**characterized in that**
the conduit means (310) extends through a conduit means duct (290) of the dispensing plunger (280) into the interior (215).

2. The device (100) according to claim 1, **wherein** the conduit means (310) and the conduit means duct (290) form a first form-fitting connection (400) between the mixing unit (200) and the reservoir (300).

3. The device (100) according to claim 2, **wherein** the reservoir (300) comprises a connecting element (360) for connecting the reservoir (300) to the mixing unit (200) via a second form-fitting connection (410).

4. The device (100) according to claim 3, **wherein** the connecting element (360) is a clasp.

5. The device (100) according to claim 3 or 4, **wherein** the pivot point (420), the first form-fitting connection (400) and the second form-fitting connection (410) form the corner points of a triangle in a side view.

6. The device (100) according to any of claims 3 to 5, **wherein** the first form-fitting connection (400), the second form-fitting connection (410) and the pivot point (420) each lie on a straight line (401, 411, 421) extending in parallel with a longitudinal axis (213) of the cartridge (210), wherein the straight lines (401, 411, 421) comprise a different straight line distance from the longitudinal axis (213) of the cartridge (210).

7. The device (100) according to any of claims 3 to 6, **wherein** the second form-fitting connection (410) is formed between the connecting element (360) and the mixing rod (230).

8. The device (100) according to any of the preceding claims, **wherein** a vacuum connection (260) is arranged on the dispensing plunger (280), via which vacuum connection the interior (215) can be fluid-conductingly connected to a negative-pressure source.

9. The device (100) according to any of the preceding claims, **wherein** the conduit means (310) is a pipe and/or a tube.

10. The device (100) according to claim 9, **wherein** the conduit means (310) is axially displaceable in the conduit means duct (290).

11. The device (100) according to claim 9 or 10, **wherein** the conduit means (310) extends at least over 70% of an axial interior length (216) of the interior (215).

12. The device (100) according to any of the preceding claims, **wherein** the mixing unit (200) comprises a duct closure (270) for fluid-conductingly sealing the conduit means duct (290) after removal of the conduit means (310).

13. The device (100) according to any of the preceding claims, **wherein** the cartridge (210) comprises, at a distal cartridge end (212) axially opposite the proximal cartridge end (211), a cartridge head (220) which fluid-conductingly seals the distal cartridge end (212), wherein the cartridge head (220) comprises a cartridge head duct (225) for fluid-conductingly connecting a discharge nozzle (530) via which the bone cement paste (520) can be discharged from the interior (215) after preparation, and wherein the cartridge (210) and the cartridge head (220) are formed in one piece.

14. A method (600) for preparing a bone cement paste (520) consisting of two starting components by means of a device (100) according to any of the preceding claims, comprising the steps of:
a. fluid-conductingly opening (610) the at least one ampoule (330) by tilting the ampoule (330) stored in the reservoir container (320) about the pivot point (420) against the connection (350),
b. flowing (620) of the monomer liquid (510) from the at least one ampoule (330) into the cavity (340),
c. conveying (630) the monomer liquid (510) from the cavity (340) via the conduit means (310) into the interior (215).

15. The method (600) according to claim 14 by means of a device (100) according to any of claims 3 to 13, additionally comprising the steps of:
d. releasing (640) the second form-fitting connection (410),
e. releasing (650) the first form-fitting connection (400) by pulling the conduit means (310) out of the conduit means duct (290),
f. fluid-conductingly sealing (660) the conduit means duct (290),
g. mixing (670) bone cement powder (500) and monomer liquid (510).

## Revendications

1. Dispositif (100) permettant de fournir une pâte de ciment osseux (520) à partir de deux composants de départ, comprenant
une unité de mélange (200) présentant une cartouche (210) en forme de cylindre creux comportant un espace intérieur (215) dans lequel est stockée une poudre de ciment osseux (500) en tant que premier composant de départ, un piston de distribution (280) déplaçable axialement dans l'espace intérieur (215), lequel piston de distribution ferme de manière conductrice de fluide l'espace intérieur (215) au niveau d'une extrémité proximale de cartouche (211), et une tige de mélange (230) qui est guidée dans l'espace intérieur (215) par l'intermédiaire d'un passage de tige de mélange (235) du piston de distribution (280) et qui est stockée de manière mobile dans l'espace intérieur (215), et un réservoir (300) pour un liquide monomère (510) en tant que second composant de départ, comprenant un moyen de conduite (310) qui relie de manière conductrice de fluide l'espace intérieur (215) de l'unité de mélange (200) au réservoir (300),
dans lequel le réservoir (300) présente un récipient de réservoir (320) dans lequel est disposée au moins une ampoule (330) fermée de manière conductrice de fluide et comportant un corps d'ampoule (331) et une tête d'ampoule (332), et le liquide monomère (510) est stocké dans l'ampoule (330), et le réservoir présente un espace creux (340) dans la zone de la tête d'ampoule (332), dans lequel l'espace creux (340) est relié de manière conductrice de fluide au moyen de conduite (310) et comprend une région de liaison (350) vers l'ampoule (330), dans lequel la tête d'ampoule (332) est disposée, au moins dans certaines zones, dans la région de liaison (350) et le récipient de réservoir (320) comprend, au moins dans certaines sections, une zone déformable (325), de sorte qu'un basculement de l'ampoule (330) autour d'un point de rotation (420) contre la région de liaison (350) est possible,
**caractérisé en ce que**
le moyen de conduite (310) s'étend dans l'espace intérieur (215) à travers un passage de moyen de conduite (290) du piston de distribution (280).

2. Dispositif (100) selon la revendication 1, **dans lequel** le moyen de conduite (310) et le passage de moyen de conduite (290) forment une première liaison par complémentarité de forme (400) entre l'unité de mélange (200) et le réservoir (300).

3. Dispositif (100) selon la revendication 2, **dans lequel** le réservoir (300) présente un élément de liaison (360) afin de relier le réservoir (300) à l'unité de mélange (200) par l'intermédiaire d'une seconde liaison par complémentarité de forme (410).

4. Dispositif (100) selon la revendication 3, **dans lequel** l'élément de liaison (360) est un ensemble formant agrafe.

5. Dispositif (100) selon la revendication 3 ou 4, **dans lequel** le point de rotation (420), la première liaison par complémentarité de forme (400) et la seconde liaison par complémentarité de forme (410) forment, dans une vue latérale, les sommets d'un triangle.

6. Dispositif (100) selon l'une des revendications 3 à 5, **dans lequel** la première liaison par complémentarité de forme (400), la seconde liaison par complémentarité de forme (410) et le point de rotation (420) sont respectivement situés sur une ligne droite (401, 411, 421) s'étendant parallèlement à un axe longitudinal (213) de la cartouche (210), dans lequel les lignes droites (401, 411, 421) présentent des écarts de lignes droites différents par rapport à l'axe longitudinal (213) de la cartouche (210).

7. Dispositif (100) selon l'une des revendications 3 à 6, **dans lequel** la seconde liaison par complémentarité de forme (410) est formée entre l'élément de liaison (360) et la tige de mélange (230).

8. Dispositif (100) selon l'une des revendications précédentes, **dans lequel** un raccord à vide (260) est disposé sur le piston de distribution (280), raccord à vide par l'intermédiaire duquel l'espace intérieur (215) peut être relié de manière conductrice de fluide à une source de dépression.

9. Dispositif (100) selon l'une des revendications précédentes, **dans lequel** le moyen de conduite (310) est un tube et/ou un tuyau.

10. Dispositif (100) selon la revendication 9, **dans lequel** le moyen de conduite (310) est déplaçable axialement dans le passage de moyen de conduite (290).

11. Dispositif (100) selon la revendication 9 ou 10, **dans lequel** le moyen de conduite (310) s'étend sur au moins 70 % d'une longueur axiale d'espace intérieur (216) de l'espace intérieur (215).

12. Dispositif (100) selon l'une des revendications précédentes, **dans lequel** l'unité de mélange (200) présente une fermeture de passage (270) afin de fermer de manière conductrice de fluide le passage de moyen de conduite (290) après un enlèvement du moyen de conduite (310).

13. Dispositif (100) selon l'une des revendications précédentes, **dans lequel** la cartouche (210) présente, au niveau d'une extrémité distale de cartouche (212) opposée axialement à l'extrémité proximale de cartouche (211), une tête de cartouche (220) qui ferme de manière conductrice de fluide l'extrémité distale de cartouche (212), dans lequel la tête de cartouche (220) présente un passage de tête de cartouche (225) pour la liaison de manière conductrice de fluide d'un tuba de distribution (530) par l'intermédiaire duquel la pâte de ciment osseux (520) peut être distribuée hors de l'espace intérieur (215) après la fourniture, et dans lequel la cartouche (210) et la tête de cartouche (220) sont réalisées d'une seule pièce.

14. Procédé (600) permettant de fournir une pâte de ciment osseux (520) à partir de deux composants de départ, au moyen d'un dispositif (100) selon l'une des revendications précédentes, comprenant les étapes consistant à :
a. ouvrir (610) de manière conductrice de fluide l'au moins une ampoule (330) en faisant basculer l'ampoule (330) stockée dans le récipient de réservoir (320) autour du point de rotation (420) contre la région de liaison (350),
b. laisser s'écouler (620) le liquide monomère (510) à partir de l'au moins une ampoule (330) dans l'espace creux (340),
c. acheminer (630) le liquide monomère (510) à partir de l'espace creux (340) dans l'espace intérieur (215) par l'intermédiaire du moyen de conduite (310).

15. Procédé (600) selon la revendication 14, au moyen d'un dispositif (100) selon l'une des revendications 3 à 13, comprenant en outre les étapes consistant à :
d. desserrer (640) la seconde liaison par complémentarité de forme (410),
e. desserrer (650) la première liaison par complémentarité de forme (400) en retirant le moyen de conduite (310) du passage de moyen de conduite (290),
f. fermer (660) de manière conductrice de fluide le passage de moyen de conduite (290),
g. mélanger (670) la poudre de ciment osseux (500) et le liquide monomère (510).
